# EUROPEAN PATENT SPECIFICATION

(11) **EP 3 980 089 B1**
(45) Date of publication and mention of the grant of the patent: **15.07.2026**
(21) Application number: 20817915.0
(22) Date of filing: 04.06.2020
(51) Int. Cl.: A61M 25/00, A61M 25/04, A61M 60/13, A61M 60/237, A61M 60/414, A61M 60/804, A61M 60/808, A61M 60/81, A61M 60/857

(54) **CATHETER BLOOD PUMPS AND METHODS OF USE AND MANUFACTURE**
KATHETERBLUTPUMPEN UND VERFAHREN ZUR VERWENDUNG UND HERSTELLUNG
POMPES D'ASSISTANCE CIRCULATOIRE DE CATHÉTER ET PROCÉDÉS D'UTILISATION ET DE FABRICATION

(30) Priority: 05.06.2019 US 201962857694 P
(43) Date of publication of application: 13.04.2022
(60) Divisional application: 26180979.2
(73) Proprietor: Supira Medical, Inc., Los Gatos, California 95032 (US)
(72) Inventor: HILDEBRAND, Daniel, Campbell, CA 95008 (US); BRANDT, Brian, Campbell, CA 95008 (US); CALOMENI, Michael, Campbell, CA 95008 (US); ROBINSON, Janine, Campbell, CA 95008 (US)
(74) Representative: J A Kemp LLP
(86) International application number: PCT/US2020/036097
(87) International publication number: WO 2020/247612

(56) References cited:
- WO-A1-2018/096531
- WO-A1-2018/226991
- US-A1- 2012 172 655
- US-A1- 2013 303 831
- US-A1- 2019 143 018
- US-A1- 2019 143 018

## Description

### BACKGROUND

Patients with heart disease can have severely compromised ability to drive blood flow through the heart and vasculature, presenting for example substantial risks during corrective procedures such as balloon angioplasty and stent delivery. There is a need for ways to improve the volume or stability of cardiac outflow for these patients, especially during corrective procedures.

Intra-aortic balloon pumps (IABP) are commonly used to support circulatory function, such as treating heart failure patients. Use of IABPs is common for treatment of heart failure patients, such as supporting a patient during high-risk percutaneous coronary intervention (HRPCI), stabilizing patient blood flow after cardiogenic shock, treating a patient associated with acute myocardial infarction (AMI) or treating decompensated heart failure. Such circulatory support may be used alone or in with pharmacological treatment.

An IABP commonly works by being placed within the aorta and being inflated and deflated in counterpulsation fashion with the heart contractions, and one of the functions is to attempt to provide additive support to the circulatory system.

More recently, minimally-invasive rotary blood pumps have been developed that can be inserted into the body in connection with the cardiovascular system, such as pumping arterial blood from the left ventricle into the aorta to add to the native blood pumping ability of the left side of the patient's heart. Another known method is to pump venous blood from the right ventricle to the pulmonary artery to add to the native blood pumping ability of the right side of the patient's heart. An overall goal is to reduce the workload on the patient's heart muscle to stabilize the patient, such as during a medical procedure that may put additional stress on the heart, to stabilize the patient prior to heart transplant, or for continuing support of the patient.

The smallest rotary blood pumps currently available can be percutaneously inserted into the vasculature of a patient through an access sheath, thereby not requiring surgical intervention, or through a vascular access graft. A description of this type of device is a percutaneously-inserted ventricular support device. US 2019/143,018 A1 discloses a catheter blood pump with rigid baskets around the impellers to control stiffness as background art to the invention.

There is a need to provide additional improvements to the field of ventricular support devices and similar blood pumps for treating compromised cardiac blood flow.

### SUMMARY OF THE DISCLOSURE

The disclosure herein is related to catheter blood pumps and methods of use and manufacture. The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

### BRIEF DESCRIPTION OF THE DRAWINGS

Figure 1 is a side view of an exemplary pump that includes an expandable and collapsible shroud and a plurality of expandable and collapsible impellers.
Figure 2 is a side view of an exemplary pump that includes an expandable and collapsible shroud and a plurality of expandable and collapsible impellers, where the shroud includes axially spaced scaffolds.
Figures 3A, 3B, 3C and 3D illustrate an exemplary pump that includes an expandable and collapsible shroud and a plurality of expandable and collapsible impellers, where the shroud includes axially spaced scaffolds.
Figure 4 illustrates an exemplary placement of a catheter pump.
Figure 5 illustrates a pump portion with a plurality of impellers.
Figure 6 shows a side view of a pump that includes a central, or intermediate, member axially spaced between first and second impellers.
Figures 7A-D illustrate an exemplary pump portion that includes an expandable housing or shroud, including an exemplary scaffold configuration.
Figure 8 illustrates an exemplary scaffold configuration.
Figure 9 illustrates an exemplary scaffold configuration.
Figure 10 illustrates an exemplary scaffold configuration.
Figures 11A-11F illustrate an exemplary method of positioning an exemplary blood pump.
Figures 12A and 12B illustrate portions of an exemplary shroud, where the shroud may include a polymeric scaffold.
Figures 13A and 13B illustrate portions of an exemplary shroud, where the shroud may include a polymeric scaffold.
Figure 14 illustrates an exemplary end of a shroud, including struts extending therefrom.
Figure 15 illustrates a portion of a catheter pump that includes a first and second expandable scaffold portions, the two portions having different greatest radial dimensions, with a pump outflow in between the two portions.

### DETAILED DESCRIPTION

The present disclosure is related to medical devices, systems, and methods of use and manufacture. Medical devices herein may include a distal pump portion (which may also be referred to herein as a working portion) adapted to be disposed within a physiologic vessel, wherein the distal pump portion includes one or more components that act upon fluid. For example, distal pump portions herein may include one or more rotating members that when rotated, can facilitate the movement of a fluid such as blood.

Any of the disclosure herein relating to an aspect of a system, device, or method of use can be incorporated with any other suitable disclosure herein. For example, a figure describing only one aspect of a device or method can be included with other embodiments even if that is not specifically stated in a description of one or both parts of the disclosure. It is thus understood that combinations of different portions of this disclosure are included herein unless specifically indicated otherwise.

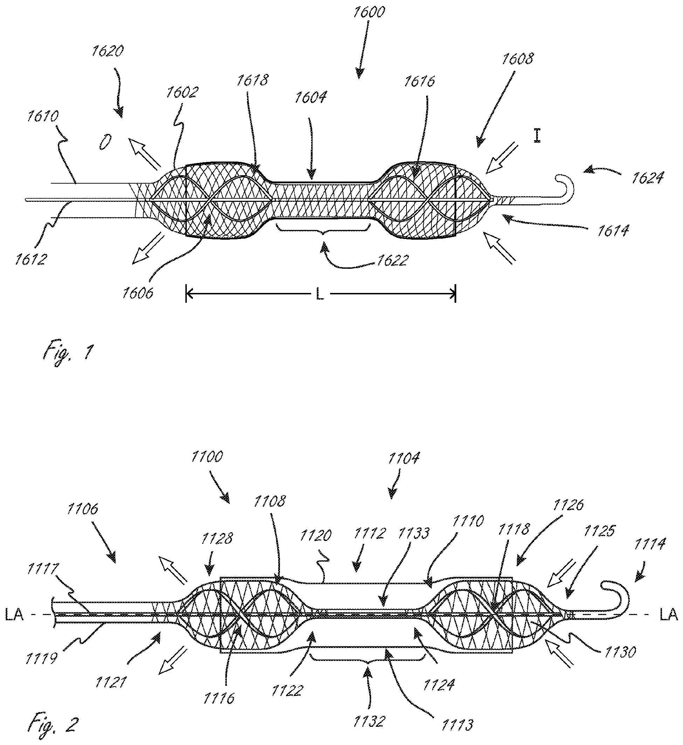

Figure 1 is a side view illustrating a distal portion of an exemplary intravascular fluid pump, including pump portion 1600, wherein pump portion 1600 includes proximal impeller 1606 and distal impeller 1616, both of which are in operable communication with drive cable 1612. Pump portion 1600 is in an expanded configuration in Figure 1, but is adapted to be collapsed to a delivery configuration so that it can be delivered with a lower profile. The impellers can be attached to drive cable 1612. Drive cable 1612 is in operable communication with an external motor, not shown, and extends through elongate shaft 1610. The phrases "pump portion" and "working portion" (or derivatives thereof) may be used herein interchangeably unless indicated to the contrary. For example without limitation, "pump portion" 1600 can also be referred to herein as a "working portion."

Pump portion 1600 also includes expandable member 1602, which in this embodiment has a proximal end 1620 that extends further proximally than a proximal end of proximal impeller 1606, and a distal end 1608 that extends further distally than a distal end 1614 of distal impeller 1616. Expandable member 1602 is disposed radially outside of the impellers along the axial length of the impellers. Expandable member 1602 can be constructed in a manner and made from materials similar to many types of expandable structures that are known in the medical arts to be able to collapsed and expanded, examples of which are provided herein. Examples of suitable materials include, but are not limited to, polyurethane and polyurethane elastomers.

Pump portion 1600 also includes conduit 1604, which is coupled to expandable member 1602, has a length L, and extends axially between the impellers. Conduit 1604 creates and provides a fluid lumen between the two impellers. When in use, fluid move through the lumen provided by conduit 1604. The conduits herein are non-permeable, or they can be semipermeable, or even porous as long as they can still define a lumen. The conduits herein are also flexible, unless it is otherwise indicated. The conduits herein extend completely around (i.e., 360 degrees) at least a portion of the pump portion. In pump portion 1600, conduit extends completely around expandable member 1602, but does not extend all the way to the proximal end 1602 or distal end 1608 of expandable member 1602. The structure of the expandable member creates at least one inlet aperture to allow for inflow "I," and at least one outflow aperture to allow for outflow "O." Conduit 1604 improves impeller pumping dynamics, compared to those that working portion 1600 would have without the conduit.

Expandable member 1602 can have a variety of constructions, and made from a variety of materials. For example, expandable member 1602 may be formed similar to expandable stents or stent-like devices, or any other example provided herein. For example without limitation, expandable member 1602 could have an open-braided construction, such as a 24-end braid, although more or fewer braid wires could be used. Exemplary materials for the expandable member include nitinol, cobalt alloys, and polymers, although other materials could be used. Expandable member 1602 has an expanded configuration, as shown, in which the outer dimension (measured orthogonally relative a longitudinal axis of the working portion) of the expandable member is greater in at least a region where it is disposed radially outside of the impellers than in a central region 1622 of the expandable member that extends axially between the impeller. Drive cable 1612 is co-axial with the longitudinal axis in this embodiment. In use, the central region can be placed across a valve, such as an aortic valve. In some embodiments, expandable member 1602 is adapted and constructed to expand to an outermost dimension of 12-24F (4.0-8.0mm) where the impellers are axially within the expandable member, and to an outermost dimension of 10-20F (3.3-6.7mm) in central region 1622 between the impellers. The smaller central region outer dimension can reduce forces acting on the valve, which can reduce or minimize damage to the valve. The larger dimensions of the expandable member in the regions of the impellers can help stabilize the working portion axially when in use. Expandable member 1602 has a general dumbbell configuration. Expandable member 1602 has an outer configuration that tapers as it transitions from the impeller regions to central region 1622, and again tapers at the distal and proximal ends of expandable member 1602.

Expandable member 1602 has a proximal end 1620 that is coupled to shaft 1610, and a distal end 1608 that is coupled to distal tip 1624. The impellers and drive cable 1612 rotate within the expandable member and conduit assembly. Drive cable 1612 is axially stabilized with respect to distal tip 1624, but is free to rotate with respect to tip 1624.

In some embodiments, expandable member 1602 can be collapsed by pulling tension from end-to-end on the expandable member. This may include linear motion (such as, for example without limitation, 5-20mm of travel) to axially extend expandable member 1602 to a collapsed configuration with collapsed outer dimension(s). Expandable member 1602 can also be collapsed by pushing an outer shaft such as a sheath over the expandable member/conduit assembly, causing the expandable member and conduit to collapse towards their collapsed delivery configuration.

Impellers 1606 and 1616 are also adapted and constructed such that one or more blades will stretch or radially compress to a reduced outermost dimension (measured orthogonally to the longitudinal axis of the working portion). For example without limitation, any of the impellers herein can include one or more blades made from a plastic formulation with spring characteristics, such as any of the impellers described in U.S. Pat. No. 7,393,181. Alternatively, for example, one or more collapsible impellers can comprise a superelastic wire frame, with polymer or other material that acts as a webbing across the wire frame, such as those described in U.S. Pat. No. 6,533,716.

The inflow and/or outflow configurations of working portion 1600 can be mostly axial in nature.

Exemplary sheathing and unsheathing techniques and concepts to collapse and expand medical devices are known, such as, for example, those described and shown in U.S. Pat. No. 7,841,976 or U.S. Pat. No. 8,052,749.

Figure 2 is a side view illustrating a deployed configuration (shown extracorporally) of a distal portion of an exemplary embodiment of a fluid movement system. Exemplary system 1100 includes working portion 1104 (which as set forth herein may also be referred to herein as a pump portion) and an elongate portion 1106 extending from working portion 1104. Elongate portion 1106 can extend to a more proximal region of the system, not shown for clarity, and that can include, for example, a motor. Working portion 1104 includes first expandable member 1108 and second expandable member 1110, axially spaced apart along a longitudinal axis LA of working portion 1104. Spaced axially in this context refers to the entire first expandable member being axially spaced from the entire second expandable member along a longitudinal axis LA of working portion 1104. A first end 1122 of first expandable member 1108 is axially spaced from a first end 1124 of second expandable member 1110.

First and second expandable members 1108 and 1110 generally each include a plurality of elongate segments disposed relative to one another to define a plurality of apertures 1130, only one of which is labeled in the second expandable member 1110. The expandable members can have a wide variety of configurations and can be constructed in a wide variety of ways, such as any of the configurations or constructions in, for example without limitation, U.S. Pat. No. 7,841,976, or the tube in 6,533,716, which is described as a self-expanding metal endoprosthetic material. For example, without limitation, one or both of the expandable members can have a braided construction or can be at least partially formed by laser cutting a tubular element.

Working portion 1104 also includes conduit 1112 that is coupled to first expandable member 1108 and to second expandable member 1110, and extends axially in between first expandable member 1108 and second expandable member 1110 in the deployed configuration. A central region 1113 of conduit 1112 spans an axial distance 1132 where the working portion is void of first and second expandable members 1108 and 1110. Central region 1113 can be considered to be axially in between the expandable members. Distal end 1126 of conduit 1112 does not extend as far distally as a distal end 1125 of second expandable member 1110, and proximal end of conduit 1128 does not extend as far proximally as proximal end 1121 of first expandable member 1108.

When the disclosure herein refers to a conduit being coupled to an expandable member, the term coupled in this context does not require that the conduit be directly attached to the expandable member so that conduit physically contacts the expandable member. Even if not directly attached, however, the term coupled in this context refers to the conduit and the expandable member being joined together such that as the expandable member expands or collapses, the conduit also begins to transition to a different configuration and/or size. Coupled in this context therefore refers to conduits that will move when the expandable member to which it is coupled transitions between expanded and collapsed configurations.

Any of the conduits herein can be deformable to some extent. For example, conduit 1112 includes elongate member 1120 that can be made of one or more materials that allow the central region 1113 of conduit to deform to some extent radially inward (towards LA) in response to, for example and when in use, forces from valve tissue (e.g., leaflets) or a replacement valve as working portion 1104 is deployed towards the configuration shown in figure 2. The conduit may be stretched tightly between the expandable members in some embodiments. The conduit may alternatively be designed with a looseness that causes a greater degree of compliance. This can be desirable when the working portion is disposed across fragile structures such as an aortic valve, which may allow the valve to compress the conduit in a way that minimizes point stresses in the valve. In some embodiments, the conduit may include a membrane attached to the proximal and distal expandable members. Exemplary materials that can be used for any conduits herein include, without limitations, polyurethane rubber, silicone rubber, acrylic rubber, expanded polytetrafluoroethylene, polyethylene, polyethylene terephthalate, including any combination thereof.

Any of the conduits herein can have a thickness of, for example, .5 - 20 thousandths of an inch (thou), such as 1-15 thou, or 1.5 to 15 thou, 1.5 to 10 thou, or 2 to 10 thou.

Any of the conduits herein, or at least a portion of the conduit, can be impermeable to blood. In figure 2, working portion 1104 includes a lumen that extends from distal end 1126 of conduit 1112 and extends to proximal end 1128 of conduit 1112. The lumen is defined by conduit 1112 in central region 1113, but can be thought of being defined by both the conduit and portions of the expandable members in regions axially adjacent to central region 1113. In this embodiment, however, it is the conduit material that causes the lumen to exist and prevents blood from passing through the conduit.

Any of the conduits herein that are secured to one or more expandable members can be, unless indicated to the contrary, secured so that the conduit is disposed radially outside of one or more expandable members, radially inside of one or more expandable members, or both, and the expandable member can be impregnated with the conduit material.

The proximal and distal expandable members help maintain the conduit in an open configuration to create the lumen, while each also creates a working environment for an impeller, described below. Each of the expandable members, when in the deployed configuration, is maintained in a spaced relationship relative to a respective impeller, which allows the impeller to rotate within the expandable member without contacting the expandable member. Working portion 1104 includes first impeller 1116 and second impeller 1118, with first impeller 1116 disposed radially within first expandable member 1108 and second impeller 1118 disposed radially within second expandable member 1110. In this embodiment, the two impellers even though they are distinct and separate impellers, are in operable communication with a common drive mechanism (e.g., drive cable 1117), such that when the drive mechanism is activated the two impellers rotate together. In this deployed configuration, impellers 1116 and 1118 are axially spaced apart along longitudinal axis LA, just as are the expandable members 1108 and 1110 are axially spaced apart.

Impellers 1116 and 1118 are also axially within the ends of expandable members 1108 and 1110, respectively (in addition to being radially within expandable members 1108 and 1110). The impellers herein can be considered to be axially within an expandable member even if the expandable member includes struts extending from a central region of the expandable member towards a longitudinal axis of the working portion (e.g., tapering struts in a side view). In figure 2, second expandable member 1110 extends from first end 1124 (proximal end) to second end 1125 (distal end).

In figure 2, a distal portion of impeller 1118 extends distally beyond distal end 1126 of conduit 1112, and a proximal portion of impeller 1116 extends proximally beyond proximal end 1128 of conduit 1112. In this figure, portions of each impeller are axially within the conduit in this deployed configuration.

In the exemplary embodiment shown in figure 2, impellers 1116 and 1118 are in operable communication with a common drive mechanism 1117, and in this embodiment, the impellers are each coupled to drive mechanism 1117, which extends through shaft 1119 and working portion 1104. Drive mechanism 1117 can be, for example, an elongate drive cable, which when rotated causes the impellers to rotate. In this example, as shown, drive mechanism 1117 extends to and is axially fixed relative to distal tip 1114, although it is adapted to rotate relative to distal tip 1114 when actuated. Thus, in this embodiment, the impellers and drive mechanism 1117 rotate together when the drive mechanism is rotated. Any number of known mechanisms can be used to rotate drive mechanism, such as with a motor (e.g., an external motor).

The expandable members and the conduit are not in rotational operable communication with the impellers and the drive mechanism. In this embodiment, proximal end 1121 of proximal expandable member 1108 is coupled to shaft 1119, which may be a shaft of elongate portion 1106 (e.g., an outer catheter shaft). Distal end 1122 of proximal expandable member 1108 is coupled to central tubular member 1133, through which drive mechanism 1117 extends. Central tubular member 1133 extends distally from proximal expandable member 1108 within conduit 1112 and is also coupled to proximal end 1124 of distal expandable member 1110. Drive mechanism 1117 thus rotates within and relative to central tubular member 1133. Central tubular member 1133 extends axially from proximal expandable member 1108 to distal expandable member 1110. Distal end 1125 of distal expandable member 1110 is coupled to distal tip 1114, as shown. Drive mechanism 1117 is adapted to rotate relative to tip 1114, but is axially fixed relative to tip 1114.

Working portion 1104 is adapted and configured to be collapsed to a smaller profile than its deployed configuration (which is shown in figure 2). This allows it to be delivered using a lower profile delivery device (smaller French size) than would be required if none of working portion 1104 was collapsible. Even if not specifically stated herein, any of the expandable members and impellers may be adapted and configured to be collapsible to some extent to a smaller delivery configuration.

The working portions herein can be collapsed to a collapsed delivery configuration using conventional techniques, such as with an outer sheath that is movable relative to the working portion (e.g., by axially moving one or both of the sheath and working portion). For example without limitation, any of the systems, devices, or methods shown in the following references may be used to facilitate the collapse of a working portions herein: U.S. Pat. No. 7841,976 or U.S. Pat. No. 8,052,749.

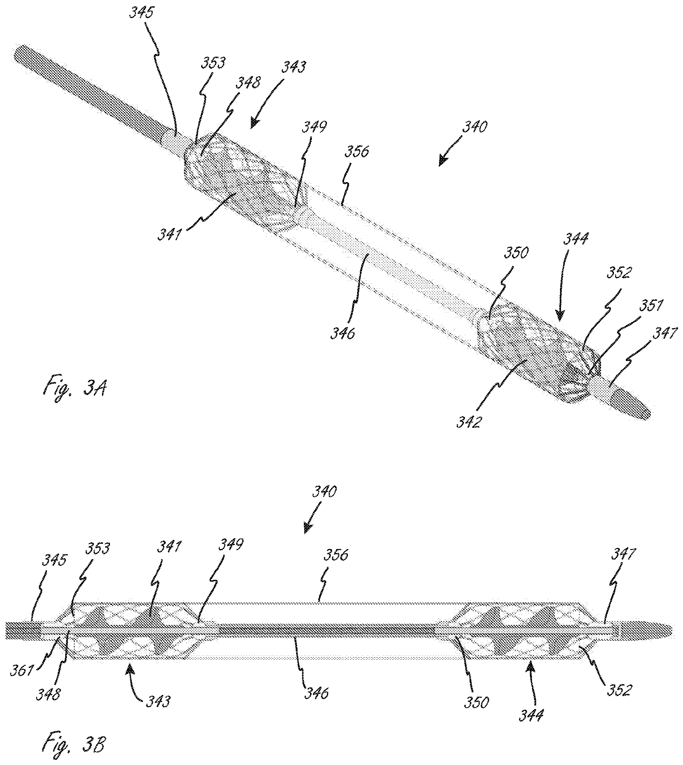

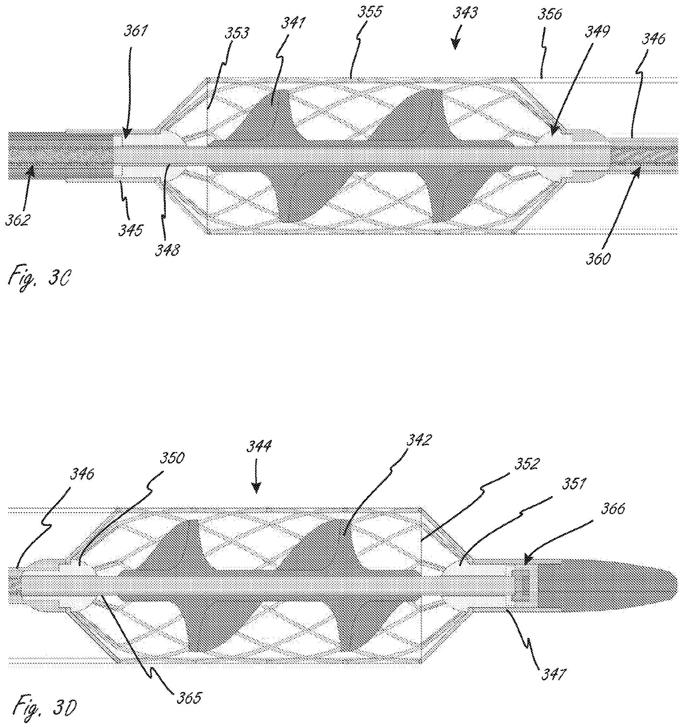

Figures 3A-3E show an exemplary working portion that is similar in some ways to the working portion shown in figure 2. Working portion 340 is similar to working portion 1104 in that it includes two expandable members axially spaced from one another when the working portion is expanded, and a conduit extending between the two expandable members. Figure 3A is a perspective view, figure 3B is a side sectional view, and figures 3C and 3D are close-up side sectional views of sections of the view in figure 3B.

Working portion 340 includes proximal impeller 341 and distal impeller 342, which are coupled to and in operational communication with a drive cable, which defines therein a lumen. The lumen can be sized to accommodate a guidewire, which can be used for delivery of the working portion to the desired location. The drive cable, in this embodiment, includes first section 362 (e.g., wound material), second section 348 (e.g., tubular member) to which proximal impeller 341 is coupled, third section 360 (e.g., wound material), and fourth section 365 (e.g., tubular material) to which distal impeller 342 is coupled. The drive cable sections all have the same inner diameter, so that lumen has a constant inner diameter. The drive cable sections can be secured to each other using known attachment techniques. A distal end of fourth section 365 extends to a distal region of the working portion, allowing the working portion to be, for example, advanced over a guidewire for positioning the working portion. In this embodiment the second and fourth sections can be stiffer than first and third sections. For example, second and fourth can be tubular and first and third sections can be wound material to impart less stiffness.

Working portion 340 includes proximal expandable member 343 and distal expandable member 344, each of which extends radially outside of one of the impellers. The expandable members have distal and proximal ends that also extend axially beyond distal and proximal ends of the impellers, which can be seen in figures 3B-3D. Coupled to the two expandable members is conduit 356, which has a proximal end 353 and a distal end 352. The two expandable members each include a plurality of proximal struts and a plurality of distal struts. The proximal struts in proximal expandable member 343 extend to and are secured to shaft section 345, which is coupled to bearing 361, through which the drive cable extends and is configured and sized to rotate. The distal struts of proximal expandable member 343 extend to and are secured to a proximal region (to a proximal end in this case) of central tubular member 346, which is disposed axially in between the expandable members. The proximal end of central tubular member 346 is coupled to bearing 349, as shown in figure 3C, through which the drive cable extends and rotates. The proximal struts of distal expandable member 344 extend to and secured to a distal region (to a distal end in this case) of central tubular member 346. Bearing 350 is also coupled to the distal region of central tubular member 346, as is shown in figure 3D. The drive cable extends through and rotates relative to bearing 350. Distal struts of distal expandable member extend to and are secured to shaft section 347 (see fig. 3A), which can be considered part of the distal tip. Shaft section 347 is coupled to bearing 351 (see fig. 3D), through which the drive cable extends and rotates relative to. The distal tip also includes bearing 366 (see figure 3D), which can be a thrust bearing. Working portion 340 can be similar to or the same in some aspects to working portion 1104, even if not explicitly included in the description. In this embodiment, conduit 356 extends at least as far as ends of the impeller, unlike in working portion 1104. Either embodiment can be modified so that the conduit extends to a position as set forth in the other embodiment. In some embodiments, section 360 can be a tubular section instead of wound.

In alternative embodiments, at least a portion of any of the impellers herein may extend outside of the fluid lumen. For example, only a portion of an impeller may extend beyond an end of the fluid lumen in either the proximal or distal direction. In some embodiments, a portion of an impeller that extends outside of the fluid lumen is a proximal portion of the impeller, and includes a proximal end (e.g., see the proximal impeller in figure 2). In some embodiments, the portion of the impeller that extends outside of the fluid lumen is a distal portion of the impeller, and includes a distal end (e.g., see the distal impeller in figure 2). When the disclosure herein refers to impellers that extend outside of the fluid lumen (or beyond an end), it is meant to refer to relative axial positions of the components, which can be most easily seen in side views or top views, such as in figure 2.

A second impeller at another end of the fluid lumen may not, however, extend beyond the fluid lumen. For example, an illustrative alternative design can include a proximal impeller that extends proximally beyond a proximal end of the fluid lumen (like the proximal impeller in figure 2), and the fluid lumen does not extend distally beyond a distal end of a distal impeller (like in figure 3B). Alternatively, a distal end of a distal impeller can extend distally beyond a distal end of the fluid lumen, but a proximal end of a proximal impeller does not extend proximally beyond a proximal end of the fluid lumen. In any of the pump portions herein, none of the impellers may extend beyond ends of the fluid lumen.

While specific exemplary locations may be shown herein, the fluid pumps may be able to be used in a variety of locations within a body. Some exemplary locations for placement include placement in the vicinity of an aortic valve or pulmonary valve, such as spanning the valve and positioned on one or both sides of the valve, and in the case of an aortic valve, optionally including a portion positioned in the ascending aorta. In some other embodiments, for example, the pumps may be, in use, positioned further downstream, such as being disposed in a descending aorta.

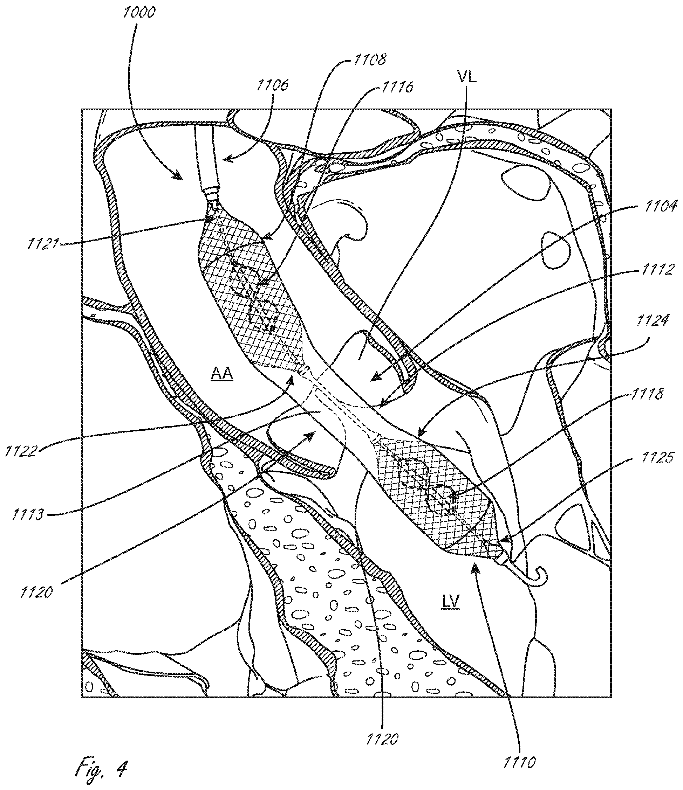

Figure 4 illustrates an exemplary placement of working portion 1104 from system 1000 from figure 2. Once difference shown in figure 4 is that the conduit extends at least as far as the ends of the impellers, like in figures 3A-3D. Figure 4 shows working portion 1104 in a deployed configuration, positioned in place across an aortic valve. Working portion 1104 can be delivered as shown via, for example without limitation, femoral artery access (a known access procedure). While not shown for clarity, system 1000 can also include an outer sheath or shaft in which working portion 1104 is disposed during delivery to a location near an aortic valve. The sheath or shaft can be moved proximally (towards the ascending aorta "AA" and away from left ventricle "LV") to allow for deployment and expansion of working portion 1104. For example, the sheath can be withdrawn to allow for expansion of second expandable member 1110, with continued proximal movement allowing first expandable member 1108 to expand.

In this embodiment, second expandable member 1110 has been expanded and positioned in a deployed configuration such that distal end 1125 is in the left ventricle "LV," and distal to aortic valve leaflets "VL," as well as distal to the annulus. Proximal end 1124 has also been positioned distal to leaflets VL, but in some methods proximal end 1124 may extend slightly axially within the leaflets VL. This embodiment is an example of a method in which at least half of the second expandable member 1110 is within the left ventricle, as measured along its length (measured along the longitudinal axis). And as shown, this is also an example of a method in which the entire second expandable member 1110 is within the left ventricle. This is also an example of a method in which at least half of second impeller 1118 is positioned within the left ventricle, and also an embodiment in which the entire second impeller 1118 is positioned within the left ventricle.

Continued retraction of an outer shaft or sheath (and/or distal movement of working end 1104 relative to an outer sheath or shaft) continues to release conduit 1112, until central region 1113 is released and deployed. The expansion of expandable members 1108 and 1110 causes conduit 1112 to assume a more open configuration, as shown in figure 4. Thus, while in this embodiment conduit 1112 does not have the same self-expanding properties as the expandable members, the conduit will assume a deployed, more open configuration when the working end is deployed. At least a portion of central region 1113 of conduit 1112 is positioned at an aortic valve coaptation region. In figures 3, there is a short length of central region 1113 that extends distally beyond the leaflets VL, but at least some portion of central region 1113 is axially within the leaflets.

Continued retraction of an outer shaft or sheath (and/or distal movement of working end 1104 relative to an outer sheath or shaft) deploys first expandable member 1108. In this embodiment, first expandable member 1108 has been expanded and positioned (as shown) in a deployed configuration such that proximal end 1121 is in the ascending aorta AA, and proximal to leaflets "VL." Distal end 1122 has also been positioned proximal to leaflets VL, but in some methods distal end 1122 may extend slightly axially within the leaflets VL. This embodiment is an example of a method in which at least half of first expandable member 1110 is within the ascending aorta, as measured along its length (measured along the longitudinal axis). And as shown, this is also an example of a method in which the entire first expandable member 1110 is within the AA. This is also an example of a method in which at least half of first impeller 1116 is positioned within the AA, and also an embodiment in which the entire first impeller 1116 is positioned within the AA.

At any time during or after deployment of working portion 1104, the position of the working portion can be assessed in any way, such as under fluoroscopy. The position of the working portion can be adjusted at any time during or after deployment. For example, after second expandable member 1110 is released but before first expandable member 1108 is released, working portion 1104 can be moved axially (distally or proximally) to reposition the working portion. Additionally, for example, the working portion can be repositioned after the entire working portion has been released from a sheath to a desired final position.

It is understood that the positions of the components (relative to the anatomy) shown in figure 4 are considered exemplary final positions for the different components of working portion 1104, even if there was repositioning that occurred after initial deployment.

The one or more expandable members herein can be configured to be, and can be expanded in a variety of ways, such as via self-expansion, mechanical actuation (e.g., one or more axially directed forces on the expandable member, expanded with a separate balloon positioned radially within the expandable member and inflated to push radially outward on the expandable member), or a combination thereof.

Expansion as used herein refers generally to reconfiguration to a larger profile with a larger radially outermost dimension (relative to the longitudinal axis), regardless of the specific manner in which the one or more components are expanded. For example, a stent that self-expands and/or is subject to a radially outward force can "expand" as that term is used herein. A device that unfurls or unrolls can also assume a larger profile, and can be considered to expand as that term is used herein.

The impellers can similarly be adapted and configured to be, and can be expanded in a variety of ways depending on their construction. For examples, one or more impellers can, upon release from a sheath, automatically revert to or towards a different larger profile configuration due to the material(s) and/or construction of the impeller design (see, for example, U.S. Pat. No. 6,533,716, or U.S. Pat. No. 7,393,181). Retraction of an outer restraint can thus, in some embodiments, allow both the expandable member and the impeller to revert naturally to a larger profile, deployed configuration without any further actuation.

As shown in the example in figure 4, the working portion includes first and second impellers that are spaced on either side of an aortic valve, each disposed within a separate expandable member. This is in contrast to some designs in which a working portion includes a single elongate expandable member. Rather than a single generally tubular expandable member extending all the way across the valve, working end 1104 includes a conduit 1112 extending between expandable members 1108 and 1110. The conduit is more flexible and deformable than the expandable baskets, which can allow for more deformation of the working portion at the location of the leaflets than would occur if an expandable member spanned the aortic valve leaflets. This can cause less damage to the leaflets after the working portion has been deployed in the subject.

Additionally, forces on a central region of a single expandable member from the leaflets might translate axially to other regions of the expandable member, perhaps causing undesired deformation of the expandable member at the locations of the one or more impellers. This may cause the outer expandable member to contact the impeller, undesirably interfering with the rotation of the impeller. Designs that include separate expandable members around each impeller, particularly where each expandable member and each impeller are supported at both ends (i.e., distal and proximal), result in a high level of precision in locating the impeller relative to the expandable member. Two separate expandable members may be able to more reliably retain their deployed configurations compared with a single expandable member.

As described herein above, it may be desirable to be able to reconfigure the working portion so that it can be delivered within a 9F sheath and still obtain high enough flow rates when in use, which is not possible with some products currently in development and/or testing. For example, some products are too large to be able to reconfigured to a small enough delivery profile, while some smaller designs may not be able to achieve the desired high flow rates. An exemplary advantage of the examples in figures 1, 2, 3A-3D and 4 is that, for example, the first and second impellers can work together to achieve the desired flow rates, and by having two axially spaced impellers, the overall working portion can be reconfigured to a smaller delivery profile than designs in which a single impeller is used to achieved the desired flow rates. These embodiments thus use a plurality of smaller, reconfigurable impellers that are axially spaced to achieve both the desired smaller delivery profile as well as to achieve the desired high flow rates.

The embodiment herein can thus achieve a smaller delivery profile while maintaining sufficiently high flow rates, while creating a more deformable and flexible central region of the working portion, the exemplary benefits of which are described above (e.g., interfacing with delicate valve leaflets).

Any of the conduits herein act to, are configured to, and are made of material(s) that create a fluid lumen therein between an first end (e.g., distal end) and a second end (e.g., proximal end). Fluid flows into the inflow region, through the fluid lumen, and then out of an outflow region. Flow into the inflow may be labeled herein as "I," and the outflow may be labeled "O" herein. Any of the conduits herein can be impermeable. Any of the conduits herein can alternatively be semipermeable. Any of the conduits herein may also be porous, but will still define a fluid lumen therethrough. In some embodiments the conduit is a membrane, or other relatively thin layered member. Any of the conduits herein, unless indicated to the contrary, can be secured to an expandable member such that the conduit, where is it secured, can be radially inside and/or outside of the expandable member. For example, a conduit can extend radially within the expandable member so that inner surface of the conduit is radially within the expandable member where it is secured to the expandable member.

Any of the expandable member(s) herein can be constructed of a variety of materials and in a variety of ways. For example, the expandable member may have a braided construction, or it can be formed by laser machining. The material can be deformable, such as nitinol. The expandable member can be self-expanding or can be adapted to be at least partially actively expanded.

In some embodiments, the expandable member is adapted to self-expand when released from within a containing tubular member such as a delivery catheter, a guide catheter or an access sheath. In some alternative embodiments, the expandable member is adapted to expand by active expansion, such as action of a pull-rod that moves at least one of the distal end and the proximal end of the expandable member toward each other. In alternative embodiments, the deployed configuration can be influenced by the configuration of one or more expandable structures. In some embodiments, the one or more expandable members can deployed, at least in part, through the influence of blood flowing through the conduit. Any combination of the above mechanisms of expansion may be used.

The blood pumps and fluid movement devices, system and methods herein can be used and positioned in a variety of locations within a body. While specific examples may be provided herein, it is understood that that the working portions can be positioned in different regions of a body than those specifically described herein.

In any of the embodiments herein, the pump portion can have a compliant or semi-compliant (referred to generally together as "compliant") exterior structure. In various embodiments, the compliant portion is pliable. In various embodiments, the compliant portion deforms only partially under pressure. For example, the central portion of the pump may be formed of a compliant exterior structure such that it deforms in response to forces of the valve. In this manner the exterior forces of the pump on the valve leaflets are reduced. This can help prevent damage to the valve at the location where it spans the valve.

One aspect of the disclosure is an intravascular blood pump that includes a distal impeller axially spaced from a proximal impeller. In one embodiment, the distal and proximal impellers are separated from each other. For example, the distal and proximal impellers may be connected solely by their individual attachment to a common driveshaft. This is distinct from an impeller having multiple blade rows. A distal impeller as that phrase is used herein does not necessarily mean a distal-most impeller of the pump, but can refer generally to an impeller that is positioned further distally than a proximal impeller, even if there is an additional impeller than is disposed further distally than the distal impeller. Similarly, a proximal impeller as that phrase is used herein does not necessarily mean a proximal-most impeller of the pump, but can refer generally to an impeller that is positioned further proximally than a proximal impeller, even if there is an additional impeller than is disposed further proximally than the proximal impeller. Axial spacing (or some derivative thereof) refers to spacing along the length of a pump portion, such as along a longitudinal axis of the pump portion, even if there is a bend in the pump portion. In various embodiments, each of the proximal and distal impellers are positioned within respective housings and configured to maintain a precise, consistent tip gap, and the span between the impellers has a relatively more flexible (or completely flexible) fluid lumen. For example, each of the impellers may be positioned within a respective housing having relatively rigid outer wall to resist radial collapse. The sections between the impellers may be relatively rigid, in some embodiments the section is held open primarily by the fluid pressure within.

In any of the embodiments herein, a tip gap exists between an impeller outer diameter and a fluid lumen inner diameter. In some embodiments the tip gap can be from 0.01 mm - 1mm, such as .05 mm to .8 mm, or such as 0.1 mm - 0.5 mm.

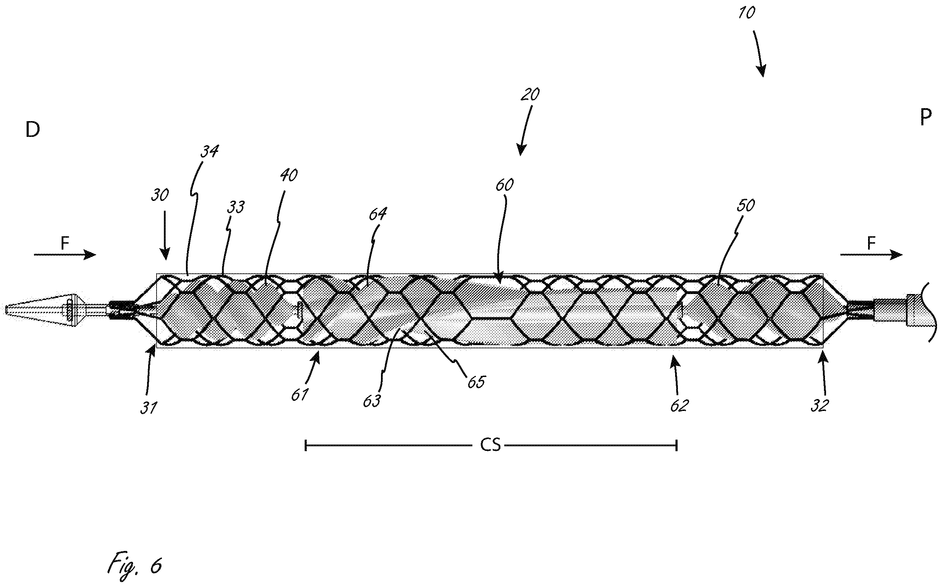

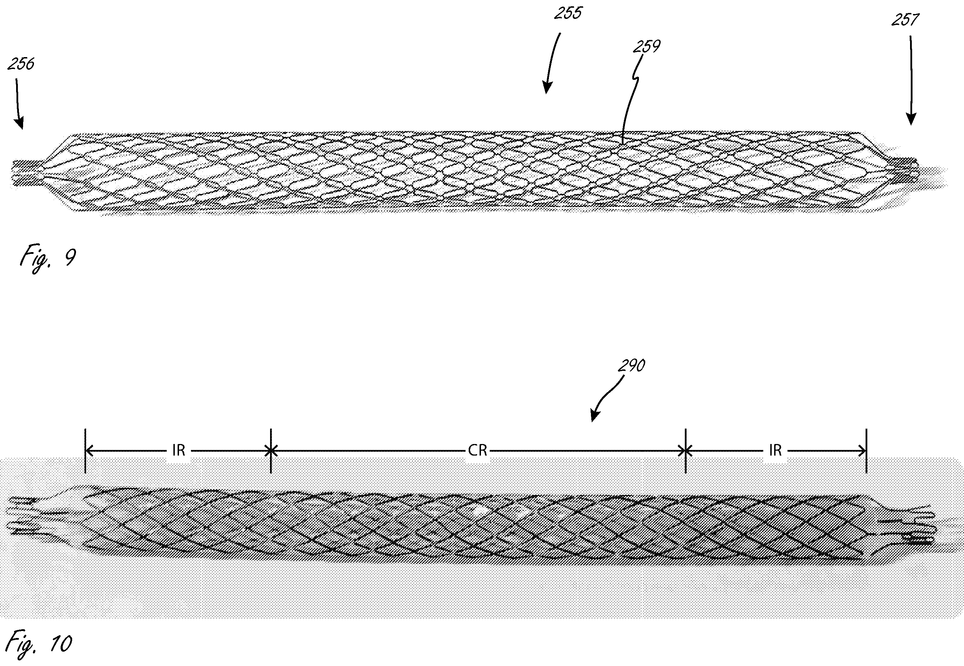

Figure 6 is a side view illustrating an exemplary distal portion 20 of a fluid pumping apparatus 10. The distal direction is indicated with a "D" and the proximal direction is indicated with a "P." Distal portion 20 (and other distal portions herein) may also be referred to as a pump portion. Distal portion 20 includes expandable member 30, which includes a conduit for fluid flow as is described herein. Expandable member 30 includes a support structure 33 (which may be referred to herein as a scaffold), which in this embodiment is a stent-like member, but can be constructed using any of the examples provided herein. Expandable member 30 also includes a membrane 34 that has a distal end 31 and proximal end 32. Membrane 34 is coupled to support structure 33. Membrane 34 at least partially creates and defines an internal lumen through which fluid flows when impellers 40 and 50 are activated. Membrane 34 can have any of the properties of any of the conduits that are described herein. When support structure 33 expands to the deployed and expanded configuration shown in figure 10, the conduit also assumes the open configuration shown in figure 10. Fluid flow is indicated generally in the direction of arrows "F" when impellers 40 and 50 are activated. Impellers 40 and 50 can be any of the impellers described herein and can have any of the properties described herein.

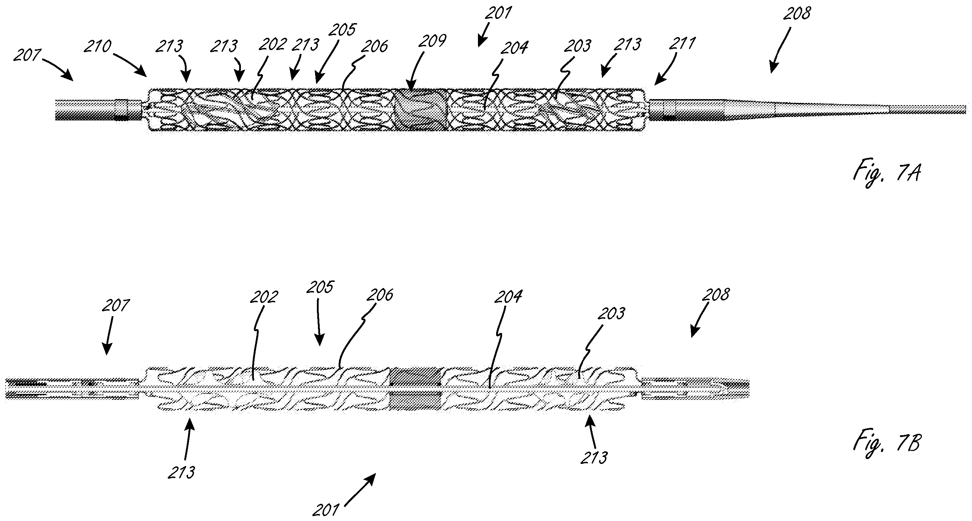

Figures 7A-7F illustrate exemplary pump portion 201 of an exemplary blood pump. Pump portion 201 can be used interchangeably with any other aspect of the any of the blood pumps herein. Figure 7A is a side view, and figure 7B is a sectional side view.

Pump portion 201 includes drive cable tubular member 204, to which distal impeller 203 and proximal impeller 202 are secured. Rotation of drive cable tubular member 204, via rotation of the drive cable (not shown), causes rotation of the impellers. More or fewer than two impellers may be included in the pump portion.

Pump portion 201 also includes a collapsible housing 205, which includes collapsible support structure 206 (which may be referred to herein as a scaffold) with proximal end 210 and distal end 211, and conduit 212 (see figure 17E), which forms a fluid lumen between a distal end and a proximal end of the fluid lumen.

Pump portion 201 includes optional intermediate (which may be referred to herein as central, or in between impellers) member 209 between two impellers, which may be any central member or members herein.

In any of the embodiments herein, the distal impeller can have a length that is less than a proximal impeller, such as is shown in the device in figure 7A.

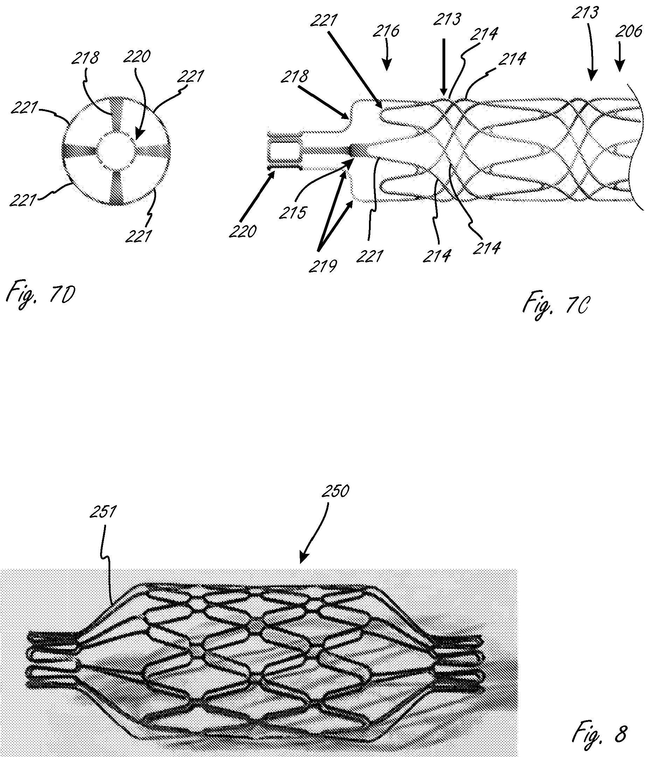

Figure 7C is a side view of a proximal portion of support structure 206 in an expanded configuration (other parts not shown for clarity). Figure 7D is a proximal end view of the support structure. The region shown is generally surrounding impeller 202 in figures 7A and B. Support structure 206 can be formed using a variety of techniques, such as laser cutting a tubular starting material. Support structure 206 includes a plurality of arms (four in this embodiment) that, at the proximal region, transition from a larger diameter to a smaller diameter in regions 218. Each of the arms has a bend in regions 219, and is vertical in between the bend regions, as shown. The vertical region can help stabilize the transition region between the larger diameter and smaller diameter regions, and reduce and preferably eliminate the influence on the fluid at the outflow.

In the larger diameter region of the support structure, the support structure 206 includes staggered peaks 221 (only two are labeled), alternating every other peak. Staggered in this context refers to the axial location of the end of the peak. Each of the four arms forms a peak that extends further proximally than adjacent peak. The staggered peaks can facilitate sheathing and offset packing volume during collapse of the pump portion. A peak as used herein may also be considered a valley depending on the orientation, similar to how convex and concave are relative terms.

Support structure 206 also includes axially spaced helical regions 213 (only some are labeled in figures 7A and 7B) that include a plurality of arms (or portions of arms) that have helical configurations. In figure 7C, helical region 213 includes helical arms 214 (only four are labeled). In this embodiment, the helical arms extend between adjacent non-helical regions of the support structure. The regions in between the helical regions can have any number of configurations, and exemplary configurations are shown. In this exemplary embodiment, proximal impeller 202 axially overlaps with a least a portion of two adjacent helical regions 213, and distal impeller axially overlaps with at least a portion of two adjacent helical regions 213. Any impeller can axially overlap with one or more helical section 213. The pitch of the helical arms can vary.

Figure 8 illustrates an expandable member 250 that is one of at least two expandable members (which may also be referred herein as collapsible housings), such as the expandable members in figures 3A-3D, wherein each expandable member surrounds an impeller. The scaffold design in figure 8 has more proximal struts 251 (only one labeled) than the design in figures 7A-7D (in this exemplary embodiment there are nine compared to four). Having a separate expandable member 250 for each impeller provides for the ability to have very different geometries for any of the individual impellers. Additionally, this design reduces the amount of scaffold material (e.g., Nitinol) over the length of the scaffold (compared to other full length scaffolds herein), which may offer increased tracking when sheathed). A potential challenge with this design may include creating a continuous membrane between the expandable members in the absence of an axially extending scaffolding material (see figure 3A). Additionally, a relatively higher number of proximal struts 251 in the outflow path may disrupt the outflow more than designs with fewer numbers of struts, such as the four struts in the embodiment in figures 7A-D. Any other aspect of the expandable member(s) herein, such as those described in figures 3A-3D, may be incorporated by reference into this exemplary design. Figure 8 shows a planar view of the scaffold in a non-expanded configuration to further illustrate the design.

Figure 9 illustrates a scaffold design that has the same general pattern as in figure 8, but the scaffold pattern is not separated into two discrete sections, but rather the scaffold is a single elongate member as shown. Figure 9 is an expanded configuration. The scaffold design in figure 9 has proximal end 256 and distal end 257 (i.e., the hub coupling regions) that have continuous, integral formations, rather than the independent proximal hub ends as in the design in figures 7A-D. It may be easier to apply (e.g., coat) a membrane to the single scaffold in this design and the design in figures 17A-D (compared to, for example, the separate axially spaced expandable members such as in figure 8). An exemplary drawback may be the relatively higher number of proximal struts (nine in this embodiment), which like the design in figure 8B may disrupt the outflow as the blood exits the fluid lumen. This particular pattern may also be too rigid for some applications or access routes where more increased bending and flexing are desired. This design is relatively rigid over the axial length, and does not bend or flex with great ease. In this design, each peak 258 and valley 260 in adjacent sections 261 are radially aligned and are coupled by connector 259, which is parallel with a longitudinal axis of the fluid lumen.

Figure 21B shows an exemplary scaffold design. A central region "CR", is between regions in which proximal and distal impellers would be located. The benefits of this increased flexibility in this region are described herein.

The scaffold may have relatively more rigid impeller sections "IR", adjacent the central region, where the impellers are disposed (not shown). The relatively increased rigidity in the impeller regions IR can help maintain tip gap and impeller concentricity. This scaffold pattern thus provides for a flexibility distribution, along its length, of a proximal section of relatively less flexibility ("IR"), a central region "CR" of relatively higher flexibility, and a distal section "IR" of relatively less flexibility. The relatively less flexibility sections (i.e., the two IR regions) are where proximal and distal impellers can be disposed (not shown but other embodiments are fully incorporated herein in this regard), with a relatively more flexible region in between. The benefits of the relative flexibility in these respective section are described elsewhere herein.

One or more impellers that are part of a blood pump system (such as any herein) may be rotated at relatively high speeds, such as between 10,000 and 50,000 RPM. Impellers can be rotated by being in rotational communication with a drive member (e.g., a drive cable) or other component in rotational communication with the impeller, which can be rotated by an energy source (e.g., motor). Rotating the drive member at the same RPMs as the impellers may cause wear on the drive member, vibration, and perhaps requires lubricating (aspects of exemplary lubricating systems are described elsewhere herein) the drive member. It may be advantageous to have the drive member rotating at speeds less than the impellers, while still causing the impellers to rotate at the desired higher RPMs. One aspect of this disclosure is a blood pump that includes one or more drive members that can be rotated at lower RPMs than one or more impellers. This may decrease drive member wear, reduce lubrication needs, and reduce vibration. This may be particularly advantageous in applications in which the blood pumps are used for relatively long terms (e.g., 24 hours or more). For example, this may be particularly advantageous for cardiogenic shock indications.

The following disclosure provides exemplary method steps that may be performed when using any of the blood pumps, or portions thereof, described herein. It is understood that not all of the steps need to be performed, but rather the steps are intended to be an illustrative procedure. It is also intended that, if suitable, in some instances the order of one or more steps may be different.

Before use, the blood pump can be prepared for use by priming the lumens (including any annular spaces) and pump assembly with sterile solution (e.g., heparinized saline) to remove any air bubbles from any fluid lines. The catheter, including any number of purge lines, may then be connected to a console. Alternatively, the catheter may be connected to a console and/or a separate pump that are used to prime the catheter to remove air bubbles.

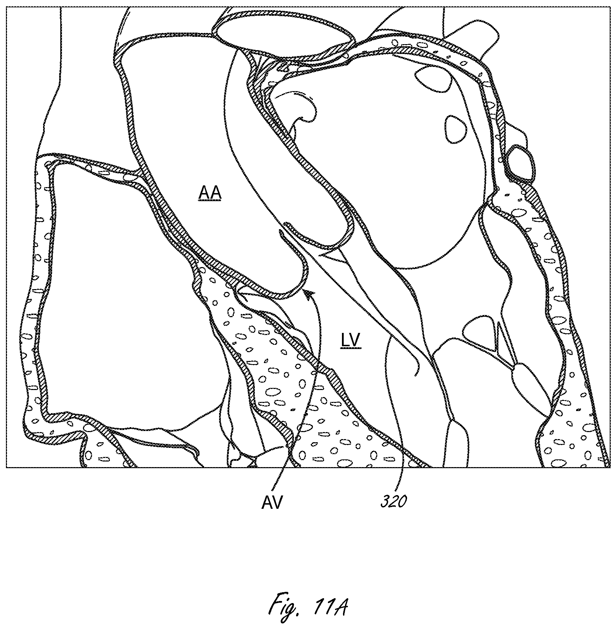

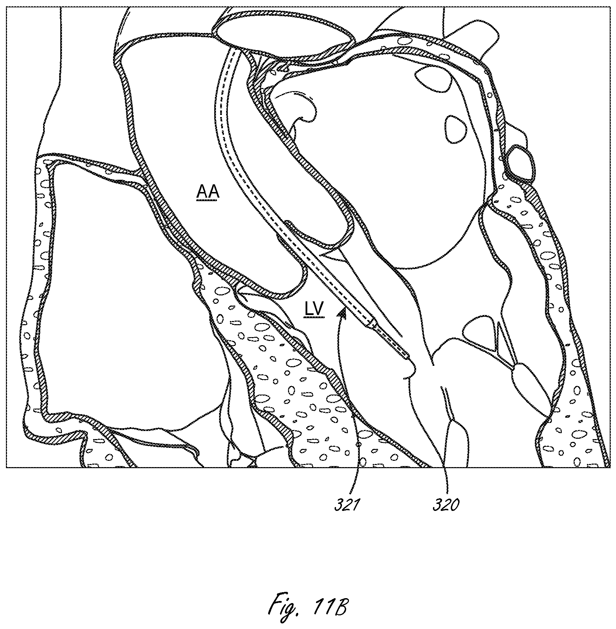

After priming the catheter, access to the patient's vasculature can be obtained (e.g., without limitation, via femoral access) using an appropriately sized introducer sheath. Using standard valve crossing techniques, a diagnostic pigtail catheter may then be advanced over a, for example, 0.035" guide wire until the pigtail catheter is positioned securely in the target location (e.g., left ventricle). The guidewire can then be removed and a second wire 320 (e.g., a 0.018" wire) can be inserted through the pigtail catheter. The pigtail catheter can then be removed (see figure 11A), and the blood pump 321 (including a catheter, catheter sheath, and pump portion within the sheath; see figure 11B) can be advanced over the second wire towards a target location, such as spanning an aortic valve "AV," and into a target location (e.g., left ventricle "LV"), using, for example, one or more radiopaque markers to position the blood pump.

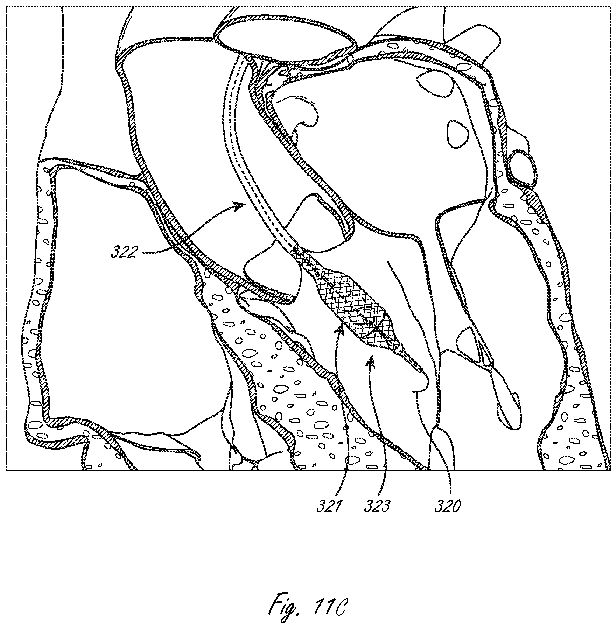

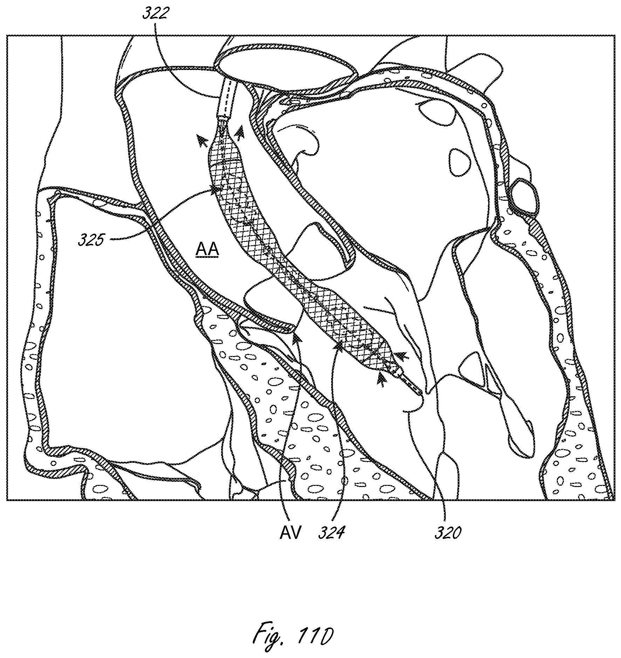

Once proper placement is confirmed, the catheter sheath 322 (see fig 11C) can be retracted, exposing first a distal region of the pump portion. In figure 11C a distal region of an expandable housing has been released from sheath 322 and is expanded, as is distal impeller 324. A proximal end of housing 323 and a proximal impeller are not yet released from sheath 322. Continued retraction of sheath 322 beyond the proximal end of housing 323 allows the housing 323 and proximal impeller 325 to expand (see figure 11D). The inflow region (shown with arrows even though the impellers are not yet rotating) and the distal impeller are in the left ventricle. The outflow (shown with arrows even though the impellers are not rotating yet) and proximal impeller are in the ascending aorta AA. The region of the outer housing in between the two impellers, which may be more flexible than the housing regions surrounding the impellers, as described in more detail herein, spans the aortic valve AV. In an exemplary operating position as shown, an inlet portion of the pump portion will be distal to the aortic valve, in the left ventricle, and an outlet of the pump portion will be proximal to the aortic valve, in the ascending aorta ("AA").

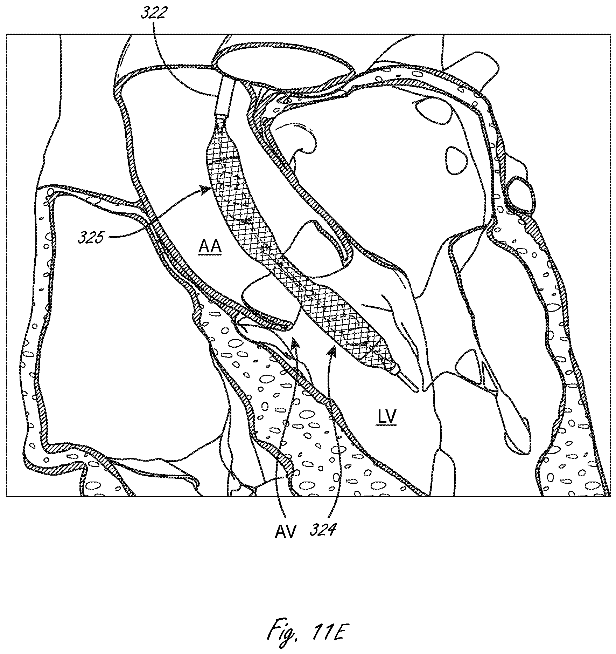

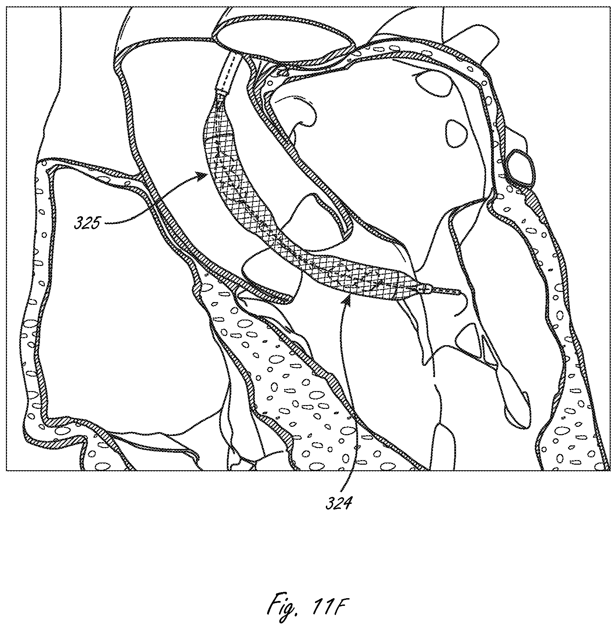

The second wire (e.g., an 0.018" guidewire) may then be moved prior to operation of the pump assembly (see figure 11E). If desired or needed, the pump portion can be deflected (active or passively) at one or more locations as described herein, as illustrated in figure 11F. For example, a region between two impellers can be deflected by tensioning a tensioning member that extends to a location between two impellers. The deflection may be desired or needed to accommodate the specific anatomy. As needed, the pump portion can be repositioned to achieve the intended placement, such as, for example, having a first impeller on one side of a heart valve and a second impeller on a second side of the heart valve. It is understood that in figure 11F, the pump portion is not in any way interfering or interacting with the mitral valve, even if it may appear that way from the figure.

In some examples herein, the pump includes one or more radial support scaffolds that are adapted to provide radial support to the blood conduit or shroud. These radial support scaffolds may be referred to herein as scaffolds, expandable members, support structures, etc., and they generally provide radial support for the expandable and collapsible fluid conduits or shrouds herein. In some instances the one or more radial support scaffolds cause the fluid conduit to assume the expanded configuration upon release from a sheath.

Some conduit support members herein are described as self-expanding material such as Nitinol. In some alternative embodiments, however, the scaffold may be polymeric rather than a metal or metal alloy such as Nitinol. Metal alloys may be referred to herein as metal, both of which may be referred to herein as metallic generally. In some embodiments the entirety, or substantially the entirety, of the expandable housing or shroud may be free of metallic material such as Nitinol. Substantially free in this context may refer to more than 90% of the shroud being free of metallic materials. The radial support may be provided by regions of polymeric material rather than metallic materials. The expandable shrouds or housings may include one or more membrane materials, and one or more generally stiffer polymeric scaffolds that provide radial support to the blood conduit at the location of the polymeric scaffolds. In general, any of the radial support scaffolds described herein (e.g., expandable members) may be polymeric scaffolds.

In some embodiments, the relatively higher stiffness of the one or more polymeric support members may be provided by utilizing relatively higher durometer material for the scaffold than for the shroud membrane material(s). In some embodiments, the relatively higher stiffness of the one or more polymeric scaffolds may comprise relatively thicker regions of polymeric material. In some embodiments the polymeric scaffolds may be thicker than a membrane as well having a higher durometer than a membrane durometer.

Polymeric scaffolds may provide exemplary benefits compared to metallic scaffolds. For example, manufacturing of the shroud may be simpler when utilizing polymeric materials, examples of which are provided herein. Additionally, polymeric scaffolds may provide for a more robust shroud construct, where delamination of the membrane may be less likely.

By way of example, the expandable members 343 and 344 in the exemplary blood pump in figure 3A may be polymeric scaffolds, which can be secured to a polymeric membrane conduit 356. This is an example of an expandable and collapsible shroud comprising first and second axially spaced polymeric scaffolds, where the first and second polymeric scaffolds are not connected to each other, but are secured relative to one another by their common coupling to membrane conduit 356. The pump may be positioned and used in the position shown in figure 4, for example. Additionally, by way of example only, the scaffold shown in figure 8 may be a polymeric scaffold. The struts 251 (as well as the distal struts not labeled) shown in figure 8 may also be polymeric or they may be metallic. If polymeric, they may be integrally formed with the cylindrical region of the polymeric scaffold. The term scaffold as used herein generally refers to the radial support for the shroud, and struts are generally considered to extend away from the shroud, even if they are integrally formed with the scaffold. Additionally, by way of example only, the scaffolds shown in figures 9 and 10 may be polymeric scaffolds. The struts extending from the generally cylindrical scaffolds may be polymeric or they may be metallic, which is described in more detail herein. Figure 10 is an example of a polymeric scaffold that extends along the entire or substantially the entire length of the expandable shroud 290.

Figure 10 illustrates exemplary regions of expandable and collapsible shroud 290. In some embodiments the pump may include first and second impellers, and "IR" refers to impeller regions of the expandable shroud. "CR" refers to a central region, and is disposed between the two impeller regions. Polymeric scaffolds herein may extend along all or a portion of the shroud. A polymeric scaffold may extend along the entire shroud length or substantially the entire shroud length (e.g., figure 10). A polymeric scaffold may be positioned at an impeller region (e.g., figure 3A). A first polymeric scaffold may be disposed at a first impeller region, and a second polymeric scaffold may be disposed at a second impeller region.

In figure 10, the different regions of the scaffold may be made of different materials. For example only, the scaffold in the impeller regions IR may be made of Nitinol, and the scaffold in the central region may be a polymeric material. This may be advantageous if, for example, the central region is desired to be more flexible than the impeller regions, but a stiffer metallic material is desired in one or both of the impeller regions. This may be advantageous if the central region may benefit from being atraumatic, such as if the central region is positioned against sensitive tissue such as an aortic heart valve. The strands or elongate elements of the different materials may connect at the junctions of the impeller regions and the central regions to form continuous strands even though not integrally formed from the same type of material. This is an example of a polymeric scaffold extending along at least a central region of the expandable shroud.

Some blood pumps may include a single impeller, which may be disposed in a proximal half or a distal half of the expandable shroud. These pumps may include any of the polymeric scaffolds herein. For example, in blood pumps that include an impeller is a distal region of the shroud, the impeller region may include a metallic scaffold, but a polymeric scaffold(s) may extend proximally from the impeller regions. For example, in blood pumps that include an impeller in a proximal region of the shroud, the impeller region may include a metallic scaffold, but a polymeric scaffold(s) may extend distally from the impeller regions.

Polymeric scaffolds can have any position and length that is desired to impart physical properties to any portion of the shroud.

Within any of the individual polymeric scaffolds herein, the stiffness of the polymeric scaffold may vary over its length. This may be beneficial if it is desirable to vary the properties of the shroud at different axial locations. Additionally, stiffness may vary gradually, as abrupt changes in stiffness may be generally less preferred along the length of the shroud. Gradual transitions in durometer, for example, may help prevent more abrupt transitions. Transitions in durometer may be formed using masking techniques. For example, a first polymeric material may be sprayed onto a desired location with adjacent regions masked. Subsequently, a second polymeric material with a different durometer may be sprayed while masking areas previously sprayed with the first durometer. This is merely an example but illustrative of creating polymeric scaffold with varying durometer along their lengths, including creating gradual changes in durometer.

Additionally, if there are two axially spaced polymeric scaffolds, such as in figure 3A, the polymeric scaffolds at the ends of the shrouds may be have different stiffness, such as by having different durometers. Additionally still, the axially spaced polymeric scaffolds may each have durometers that vary along their lengths, and the manner in which they vary along their lengths may differ. For example, one of the polymeric scaffolds may have a greater variance in durometer than the other scaffold.

In some embodiments, a polymeric scaffold extends over the entire or substantially the entire shroud length, such as figures 9 and 10 if the scaffold is polymeric. The scaffold may be stiffer in the impeller regions than it is in the central region, which may provide more radial support in the regions of the impellers, and which also may impart more flexibility in the central region. Greater stiffness in impeller regions may be imparted by higher durometer material and/or greater thickness, for example.

Any of the membranes herein may have a stiffness that is not constant along its length. For example, the membranes may have greater stiffness in the impeller regions to provide more radial support in the impeller regions than in the central region. In some embodiments, both the membrane and the scaffold may have greater stiffness (e.g., higher durometer) in the impeller regions (on average) than in the central region.

When the phrase impeller region is used herein, it does not necessarily require the entire length of the impeller, but refers to at least a portion of the shroud that surrounds an impeller. For example, an impeller region may surround a substantial portion of the impeller, but may not surround the entire impeller.

Some polymeric scaffolds herein may comprise a fabric or woven polymer elements that may be impregnated or saturated with polymer to form the laminate. In these embodiments, the polymeric scaffold may be more of an annular or cylindrical band rather than the elongate elements that are spaced further apart, as described herein. For example, a polymeric scaffold may comprise a fabric or woven polymer elements extending around the expandable shroud and extending along any length of the shroud. The fabric may be disposed at an impeller region, a central region, and/or may extend along substantially the entire shroud length.

Polymeric scaffold herein may have a higher durometer than a durometer of the membrane of the fluid conduit. Polymeric scaffolds herein may have a greater stiffness than a stiffness of the membrane. The greater stiffness may be due at least partially to the durometer of the material. In any embodiment that includes a least one polymeric scaffold, any other suitable feature (e.g., one or more impellers) or method of use herein is incorporated by reference into these embodiments.

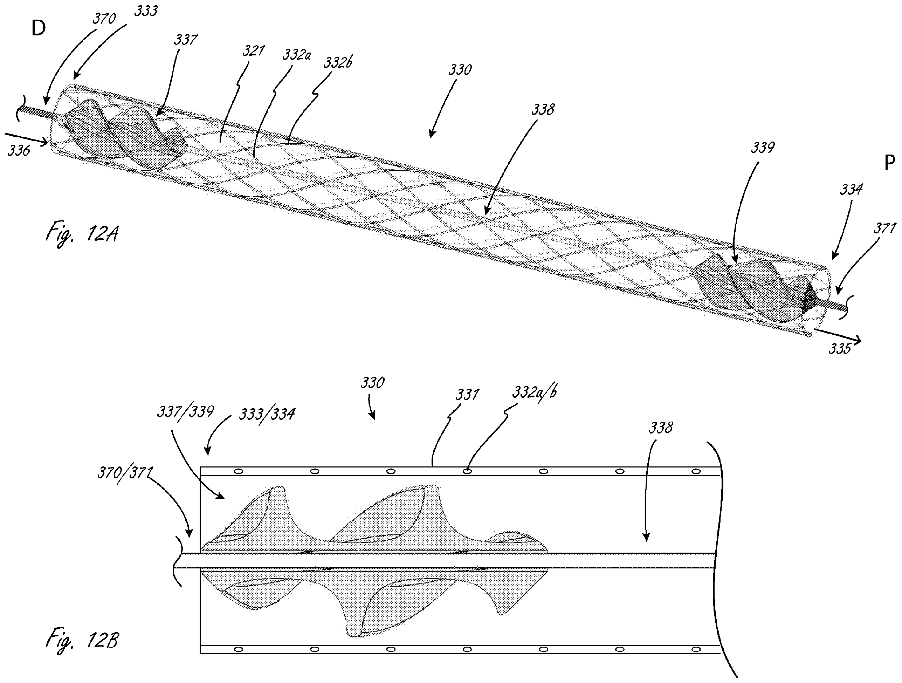

Figures 12A and 12B illustrate an exemplary expandable housing or shroud. For example, proximal and distal struts of the housing are not shown for clarity. Shroud or housing 330 includes a conduit that defines a fluid lumen having a fluid lumen distal end 333 (adjacent the inflow 336) and a fluid lumen proximal end 334 (adjacent outflow 335). Shroud 330 includes an elongate body member 331 (e.g., a membrane) and a polymeric scaffold, in this example including a plurality of polymeric elongate elements 332A and 332B. In this merely exemplary embodiment, the blood pump includes a distal impeller 337 and a proximal impeller 339, examples of which are provided herein. Other features such as a drive mechanism that includes a drive cable and coupling member 338 can be incorporated in this embodiment, even if not described. In other embodiments the pump portion can have fewer or more than two impellers. The pump may have a single impeller in a distal half of the conduit, or a single impeller in a proximal half of the conduit, for example,.

Figure 12B illustrates a partial sectional side view (showing only one impeller) of the pump in figure 12A. In this exemplary embodiment, at least some of relatively higher durometer elongate elements 332A and 332B are encapsulated within lower durometer membrane 331, as shown. While two different elongate elements are labeled as support structures 332A and 332B, it is understood that any of the plurality of elongate elements (which are helical in this exemplary embodiment) can be considered a separate elongate element, and all of the separate elongate elements can be together considered a polymeric scaffold. The elongate elements may have any other configurations, such as any of the configurations shown herein (e.g., any non-helical configuration).

Figure 12A illustrates an exemplary embodiment in which the pump includes a shroud that includes a polymeric scaffold, the polymeric scaffold comprising polymeric radial elongate elements 332A and 332B, which together may create a polymeric radial scaffold.

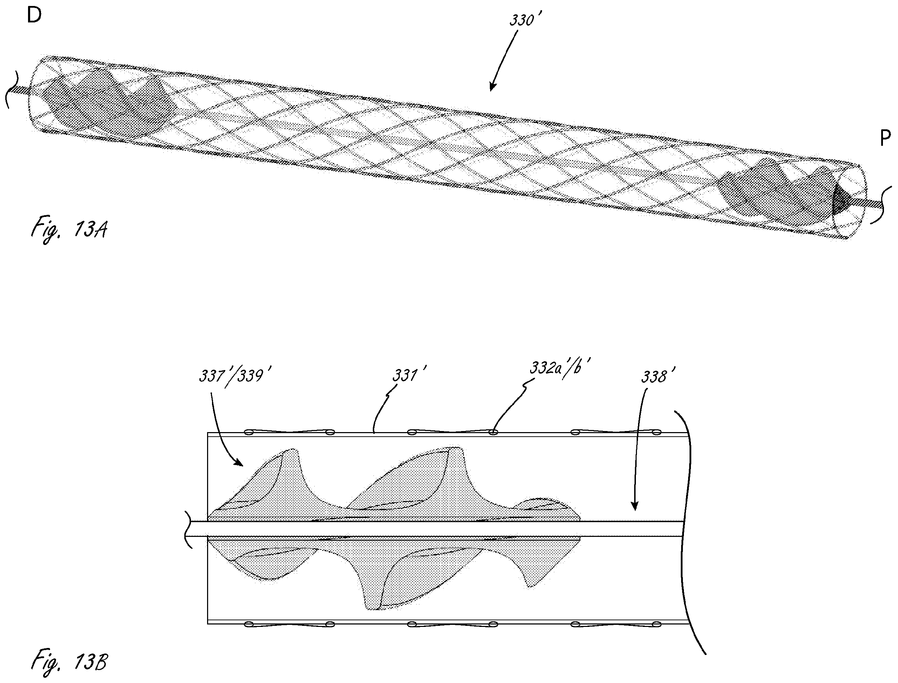

Figures 13A and 13B illustrate an embodiment of a collapsible and expandable pump shroud similar to that shown in figures 12A and 12B. The shroud 330' may be the same as the shroud in figures 29A-B in all ways not specifically mentioned herein. All reference labels in figures 13A-B are labeled with the same number but with a prime (') to indicate the features can be the same in all other regards. In figures 13A and 13B, the one or more elongate elements 332A/B' are embedded within the body member 331', rather than being encapsulated within the body member as in figures 12A and 12B. Elongate elements 332A'/B' are embedded within an outer surface of body member 331' (e.g., membrane), but alternatively they could be embedded within an inner surface of body member 331'. Alternatively, one or more elongate elements could transition from an outer surface of body member 331' to an inner surface of body member 331'.

Any of the elongate elements herein can, in alternative embodiments, be any combination of being encapsulated within or embedded within the elongate body member. For example, an elongate elements can, at some locations be embedded within an outer surface of the membrane, and in some different locations being encapsulated within the membrane, and in some locations be embedded within an inner surface of the membrane.

At least some of the polymeric scaffolds have a durometer that is greater than a durometer of at least a portion of the membrane. The higher durometer of the scaffold can help provide support to the shroud, while the lower durometer membrane generally helps provide overall desired flexibility to the shroud. In some embodiments, the scaffolds may have a durometer that is from 5D - 100 D greater than a durometer of the membrane (on average), such as 10-100 units greater (on average) on the Shore hardness scale, or 20-100 units greater (on average) on the Shore hardness scale.

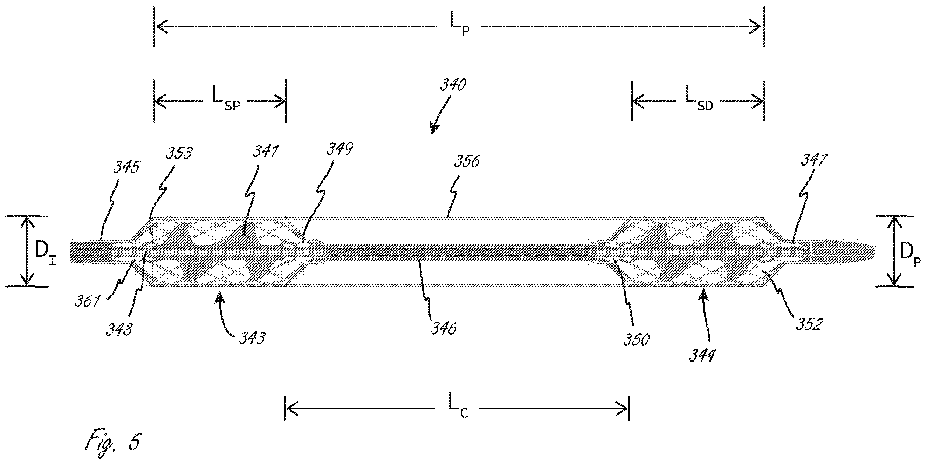

While the membranes 331 and 331' may be made from a single type of material, any of the membranes herein can be made from more than one type of material. The "type" of material as used in this context does not require a different chemical composition (but may be or include a different chemical composition), but can include a different durometer of the same material (e.g., one portion made of PEBAX 50D and one portion made of PEBAX 75D). The different materials can extend over or along a variety of different parts of the housing. For example, one type of material of an membranes can extend for less than half of the length, half the length, or more than half the length of the housing. Additionally, a second type of material can extend for less than half of the length, half the length, or more than half the length of the shroud. Additionally, for example, the membrane may be stiffer in one or more regions within which one or more impellers are disposed, such as by being made of a material with a higher durometer than axially adjacent regions (i.e., regions not radially surrounding an impeller). Increased stiffness in these regions could provide a variety of advantages, such as greater stiffness in axial regions where the impellers are located, which could help maintain tip gap between blades and the shroud. Additionally, a central region of the shroud can be made less stiff than one or more axially adjacent impeller regions by having an elongate body member with material in the central region that is less stiff (e.g., lower durometer) than axially-adjacent sections. For example, a membrane may be made less stiff (e.g., lower durometer and/or thinner) along length Lc in figure 5, which illustrates an axial spacing between impellers in this exemplary embodiment. In some embodiments that have a single impeller, the less stiff region could extend over more than just length Lc. For example, a less stiff central region may provide more flexibility in a central region, where the shroud may be positioned near delicate tissue such as a native valve tissue. Stated alternately, one or more regions of a shroud that are adjacent to a central region can be adapted with a higher stiffness than a central region, optionally with a higher durometer and/or thicker membrane.

Additionally, individual sections of the one or more scaffolds may be made from different types of material throughout the housing. For example, one or more discrete scaffolds can be made from a higher durometer than one or more other discrete support members. Or one or more scaffolds can be made from a different type of material than one or more other support member sections. For illustrative purposes only, a first scaffold may be made of a first type of material, while an adjacent scaffold can be made of a different type of material. This could be a pattern that repeats over, along, or around the shroud. Additionally or alternatively, the one or more polymeric scaffolds can have increased stiffness (e.g., due to higher durometer) at the location of an impeller, or at the location of all impellers in design where there is more than one impeller. For example, the one or more support members can have higher durometers in regions along length(s) Lsp and/or Lsd (as shown in exemplary figure 5).

In any of the embodiments herein with one or more polymeric scaffolds, one or more metallic (e.g., nitinol) support structures may also be included in one or more regions of the shroud that do or do not include polymeric scaffolds. Alternatively, a metallic scaffold may be disposed about a shroud region where an impeller is located, optionally wherein separate metallic scaffolds are disposed along two or more discrete regions where each of two or more impellers are disposed.

Expandable and collapsible shrouds with polymeric scaffolds may be manufactured using a variety of techniques. As a mere example, polymeric scaffolds may be cast or molded with dissolvable cores or cavities. If the struts the polymeric as well, the struts may be cast or molded with the polymeric scaffold. Another mere example is that polymeric scaffolds may be created by laser cutting structures (e.g., cylindrical structures) to remove material, leaving behind the scaffold structure.

And as is described above, polymeric scaffolds with durometers that vary along their lengths may be created using a variety of techniques, such as multi-shot molding, solution or spray casting, and/or masking techniques.

Coupling polymeric scaffolds to a membrane may be performed using a variety of techniques. Examples include but are not limited to: polymeric materials can be sprayed onto a formed polymeric scaffold, formed scaffolds can be dipped into the membrane material and coated, the formed polymeric scaffold could be solvent or adhesive bonded to the membrane material, or combinations thereof to form the coupled polymeric membrane and polymeric scaffold.

Any of the expandable shrouds herein may include polymeric scaffolds that comprise different material than a material of a polymeric membrane. Any of the expandable shrouds herein may comprise polymeric scaffolds that are the same material as a polymeric membrane, but the scaffold and membrane have different durometers.

Blood pumps herein may include proximal and distal struts that extend proximally and distally from the expandable shroud, wherein the proximal struts facilitate collapse of the shroud. Figure 8 illustrates struts 251, by way of example. In some embodiments in which the pump comprises one or more polymeric scaffolds, pump struts (e.g., struts 251) may be polymeric or metallic. For example, if scaffold 250 shown in figure 8 is a polymeric scaffold, struts 251 extending axially therefrom may also be polymeric. In other embodiments in which the scaffold is polymeric, the struts may be metallic, such as Nitinol.

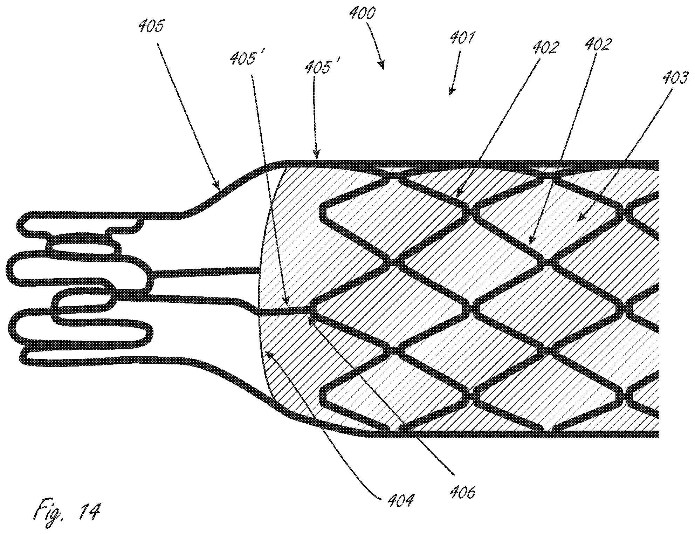

In some embodiments, the struts are metallic, such as nitinol, while the impeller regions include a polymeric scaffold. Metal struts may extend to some length into the impeller region of the shroud and may be coupled to a shroud membrane and/or polymeric scaffold. Figure 14 illustrates a portion of an exemplary collapsible and expandable shroud 401 of a pump 400, with other aspects of the pump such as a drive mechanisms and an impeller not shown for clarity. Shroud 401 includes membrane 403 and a polymeric scaffold in an impeller region. Membrane 403 has an end 404, which may be a proximal end or distal end. The polymeric scaffold includes elongate elements 402, only two sections of which are labeled. The polymeric scaffold may be any of the scaffolds herein. Pump 400 includes metallic struts 405, which have regions or sections 405' that extend into the shroud, and are coupled to membrane 403 to couple the struts 405 to the shroud 401. This is an example of metallic struts coupled to a shroud that includes a polymeric scaffold in an impeller region. While the strut regions 405' are shown as engaging portions of the polymeric scaffold at location 406, there may be some spacing between the struts and the polymeric scaffold. One or both ends of the pump shroud may be configured in the manner shown and described with respect to figure 14 (e.g., one or both of an inflow or outflow). It is understood that any other aspects of pumps herein (e.g., impeller(s)) may be incorporated with the portion of the pump in figure 14.

As is described in more detail herein, in some embodiments struts 405 may be polymeric and formed integrally with the polymeric scaffold. One or both ends of the shroud may be configured in this manner. In some embodiments, first and second impellers regions may include polymeric scaffolds that are integrally formed with polymeric struts that extend axially from the respective impeller region.

Some catheter pumps herein may include first and second expandable and collapsible scaffold sections that are axially spaced. The first and second expandable and collapsible scaffold sections have different outermost or greatest dimensions, wherein the smaller sized sections may facilitates sheathing into an outer sheath by providing a more gradual change in radial dimensions. Any other suitable feature or method described herein can be incorporated into these exemplary embodiments.

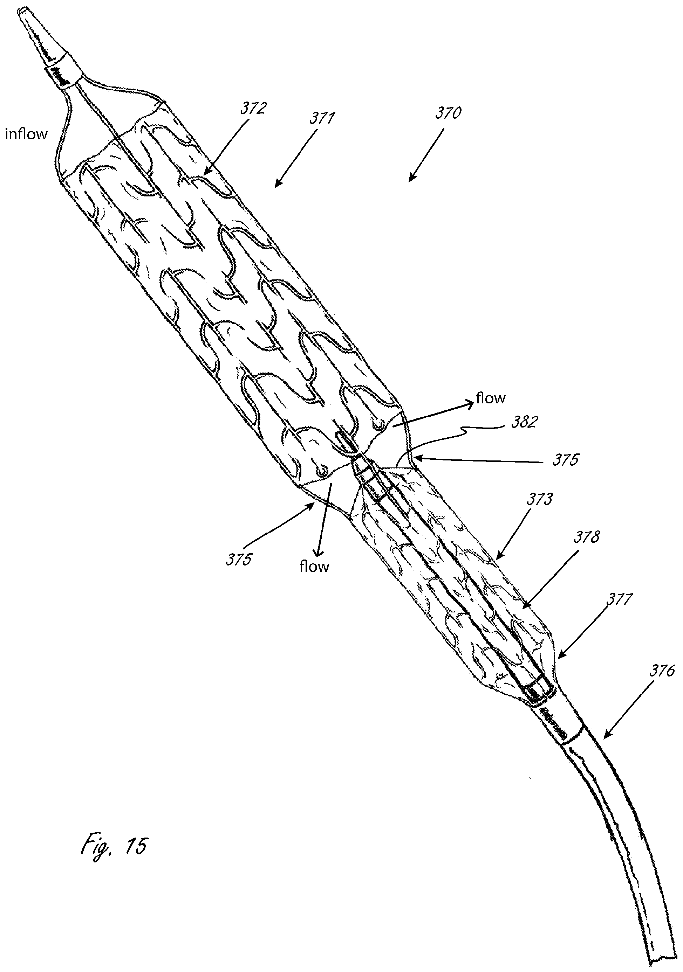

For example, figure 15 illustrates a partial view of an exemplary embodiment of a catheter blood pump including pump portion 370. Pump portion 370 includes expandable and collapsible shroud 371 that defines a blood lumen therein. The catheter pump also includes an expandable and collapsible scaffold, the scaffold comprising a first scaffold section or portion 372 with an expanded configuration and a second scaffold portion 373 with an expanded configuration, the second portion 373 axially spaced from the first portion 372. Second portion 373 has a smaller greatest outer dimension than a greatest outer dimension of the first portion 372. As shown. In this embodiment shroud 371 includes first portion 372 of the scaffold. One or more impellers may be positioned in shroud 371, which are not shown for clarity, but any of the impellers herein may be included in the example in figure 15. The outflow is labeled as Flow in figure 15. First and second scaffold portions 373 and 372 may be coupled by one or more outflow collapsible struts 373, which extend between the first and second portions 372 and 373. The catheter pump has a pump outflow in between the first and second portions, labeled as Flow in figure 15.

When shroud 371 is in an expanded configuration as shown in figure 15, second portion 373 has a greatest or outermost radial dimension (e.g., diameter) that is less than a greatest radial dimension (e.g., diameter) of the first portion 372. Greatest in this context refers to the greatest radial dimension (orthogonal to a long axis) measured in that particular section, even if the radial dimension in a particular section is not constant along its entire length. For example, tapered transition section 377 labeled may be considered part of second portion 373 even though the radial dimension is decreasing in size relative to the larger cylindrical region of second portion 373.

The reduced dimension expandable portion (e.g., second portion 373) may have a shape set configuration (which may be referred to herein as a "geometry") that is adapted to provide a more gradual reduction in dimension between shroud 371 and the diameter of shaft 376.

The outer profile of the expandable portion of the catheter pump shown in figure 15 includes two steps at which a radial dimension changes along its length. For example, the expandable portion includes a transition between first and second portions 372 and 373, which is the outflow. The catheter pump also includes transition regions 377. The "steps" result in a plurality of increases in dimension in the transition from the catheter shaft 376 to the expandable shroud 371. Both "steps" can include one or more expandable elements (e.g., struts). The stepped outer profile configuration may also facilitate sheathing of the pump portion into an outer sheath by providing a more gradual change in dimension compared with a single, possibly more abrupt change in outer radial dimension.

An impeller may have a portion extending partially outside of shroud 371, or it may be completely positioned inside the shroud 371.

Figure 15 also shows an exemplary blood flow inhibiter 382, which may also be referred to herein as a seal. In this embodiment, blood flow inhibiter can extend across the path of blood and act to prevent blood from pooling or flowing within second section 373. In this embodiment the blood flow inhibitor is secured to a shaft (optionally cylindrical) that extends through second section 373, optionally creating a seal between the blood flow inhibitor and the shaft. In some embodiments the blood flow inhibitor can include a membrane or diaphragm, or other similar relatively thin and deformable material that can function to reduce or prevent blood flow.

By way of example only, in some embodiments a first scaffold section (e.g., 372) may have a diameter from 4.5 mm - 8.5 mm (e.g., 5.5 mm - 7.5 mm), and a second scaffold section (e.g., 373) may have a diameter from 3 mm - 6.5 mm (e.g., from 4 mm - 5.5 mm).

As shown in figure 15, a plurality of struts extend between the first and second portions 372 and 373, wherein the plurality of struts are spaced and allow blood to flow therebetween at the outflow. The struts 375 are shown extending radially outward and axially towards from the second portion 373 to the first portion 372.

The catheter pump shown in figure 15 also includes membrane 378 attached to the second scaffold portion 373, similar to how shroud membranes herein are connected to shroud scaffold sections. Membrane 378 can extend all the way through transition section 377 to prevent blood from pooling or entering within the second section 373. The seal or blood flow inhibitor 382 can be an extension of the membrane, such that a membrane (which may include one or more membrane materials) covers the entire second section 373 and prevents blood from entering therein. A shaft may pass through the blood flow inhibitor 382, wherein a drive mechanism extends through the shaft and causes rotation of the one or more impellers. Depending on the delivery of the pump portion, the seal 382 may be at a distal end of the second section 373.

The catheter pump in figure 15 also includes a tapering region 377 between the second portion 373 and catheter shaft 376, wherein the tapering region is configured to facilitate collapse of the second portion 373. In some embodiments the tapering region 377 can include the second portion of the scaffold. In this example shown in figure 15, the transition region 377 includes membrane 378.

At least one of the first and second scaffold portions 372 and 373 may be polymeric, such as any of the polymeric scaffolds herein.

The first and second scaffold portions 372 and 373 may be integrally formed from the same starting material, such as nitinol, or a polymeric material. Outflow struts 375 may also be integrally formed with the first and second scaffold portions 372 and 373.

The catheter pump in figure 15 is an example of a catheter pump that includes first plurality of tapering struts (in region 377) extending in a first axial direction (e.g., distally) and radially outward, and a second plurality of tapered struts 375 extending in the first axial direction (e.g., distally) and radially outward, the first plurality of struts axially spaced from the second plurality of struts.

## Claims

1. A catheter blood pump, comprising:
an expandable and collapsible shroud (290) that defines a blood lumen, the shroud comprising a proximal impeller region (IR) and a central region (CR) positioned distally to the proximal impeller region;
a scaffold (206) extending along at least a portion of the proximal impeller region and the central region of the shroud, wherein the scaffold in the proximal impeller region has a first stiffness that is greater than a second stiffness of the scaffold in the central region, the stiffness being for providing radial support to the shroud;
a proximal impeller (20) disposed at least partially within the proximal impeller region of the shroud.

2. The blood pump of claim 1, wherein the expandable and collapsible shroud (290) comprises a distal impeller region (IR), arranged such that the central shroud region (CR) is between the distal impeller region and proximal impeller region (IR) and the stiffness is greater in the distal impeller region than in the central region.

3. The blood pump of and one of claims 1 or 2, wherein the scaffold (206) includes a plurality of helical arms (214) to define at least one helical region (213).

4. The blood pump of claim 3, wherein at least one helical region (213) axially overlaps with the proximal impeller (202).

5. The blood pump of claim 3, wherein a plurality of helical regions (213) axially overlaps with the proximal impeller (202).

6. The blood pump of claim 3, wherein at least one of the plurality of helical arms (214) extends between adjacent non-helical regions of the scaffold (206).

7. The blood pump of claim 3, wherein at least one helical region (213) extends between adjacent non-helical regions of the scaffold (206).

8. The blood pump of claim 1 or 2, wherein the proximal impeller region (IR) of the scaffold (206) includes a plurality of helical arms (214) to define at least one helical region (213).

9. The blood pump of claim 8, wherein at least one helical region (213) axially overlaps with the proximal impeller (202).

10. The blood pump of claim 8, wherein a plurality of helical regions (213) axially overlaps with the proximal impeller (202).

11. The blood pump of claim 8, wherein at least one of the plurality of helical arms (214) extends between adjacent non-helical regions of the scaffold (206).

12. The blood pump of claim 3, wherein the proximal impeller region (IR) of the scaffold (206) comprises at least one helical region (213) and the central region (CR) of the scaffold comprises at least one non-helical region.

13. The blood pump of any one of claims 1 or 2, further comprising a membrane coupled to the expandable and collapsible shroud (290) to define a fluid conduit that is impermeable to blood.

14. The blood pump of any one of claims 1 or 2, wherein the scaffold (206) comprises nitinol.

15. The blood pump of any one of claims 1 or 2, wherein the scaffold (206) comprises a polymeric structure;
wherein, optionally, a first durometer of the proximal impeller region (IR) of the scaffold is greater than a second durometer of the central region (CR) of the scaffold.

## Patentansprüche

1. Katheterblutpumpe, umfassend:
eine ausdehnbare und zusammenklappbare Ummantelung (290), die ein Blutlumen definiert, wobei die Ummantelung eine proximale Flügelradregion (IR) und eine zentrale Region (CR) umfasst, der distal zu der proximalen Flügelradregion positioniert ist;
ein Gerüst (206), das sich entlang wenigstens eines Abschnitts der proximalen Flügelradregion und der zentralen Region der Ummantelung erstreckt, wobei das Gerüst in der proximalen Flügelradregion eine erste Steifigkeit aufweist, die größer ist als eine zweite Steifigkeit des Gerüsts in der zentralen Region, wobei die Steifigkeit dazu dient, der Ummantelung eine radiale Stütze bereitzustellen;
ein proximales Flügelrad (20), das wenigstens teilweise innerhalb der proximalen Flügelradregion der Ummantelung angeordnet ist.

2. Blutpumpe nach Anspruch 1, wobei die ausdehnbare und zusammenklappbare Ummantelung (290) eine distale Flügelradregion (IR) umfasst, die derart angeordnet ist, dass sich die zentrale Ummantelungsregion (CR) zwischen der distalen Flügelradregion und der proximalen Flügelradregion (IR) befindet und die Steifigkeit in der distalen Flügelradregion größer ist als in der zentralen Region.

3. Blutpumpe nach einem der Ansprüche 1 oder 2, wobei das Gerüst (206) eine Mehrzahl von spiralförmigen Armen (214) beinhaltet, um wenigstens eine spiralförmige Region (213) zu definieren.

4. Blutpumpe nach Anspruch 3, wobei wenigstens eine spiralförmige Region (213) sich axial mit dem proximalen Flügelrad (202) überlappt.

5. Blutpumpe nach Anspruch 3, wobei eine Mehrzahl von spiralförmigen Regionen (213) sich axial mit dem proximalen Flügelrad (202) überlappt.

6. Blutpumpe nach Anspruch 3, wobei sich wenigstens einer der Mehrzahl von spiralförmigen Armen (214) zwischen benachbarten nicht spiralförmigen Regionen des Gerüsts (206) erstreckt.

7. Blutpumpe nach Anspruch 3, wobei sich wenigstens eine spiralförmige Region (213) zwischen benachbarten nicht spiralförmigen Regionen des Gerüsts (206) erstreckt.

8. Blutpumpe nach Anspruch 1 oder 2, wobei die proximale Flügelradregion (IR) des Gerüsts (206) eine Mehrzahl von spiralförmigen Armen (214) beinhaltet, um wenigstens eine spiralförmige Region (213) zu definieren.

9. Blutpumpe nach Anspruch 8, wobei sich wenigstens eine spiralförmige Region (213) axial mit dem proximalen Laufrad (202) überlappt.

10. Blutpumpe nach Anspruch 8, wobei sich eine Mehrzahl von spiralförmigen Regionen (213) axial mit dem proximalen Laufrad (202) überlappt.

11. Blutpumpe nach Anspruch 8, wobei sich wenigstens einer der Mehrzahl von spiralförmigen Armen (214) zwischen benachbarten nicht spiralförmigen Regionen des Gerüsts (206) erstreckt.

12. Blutpumpe nach Anspruch 3, wobei die proximale Laufradregion (IR) des Gerüsts (206) wenigstens eine spiralförmige Region (213) umfasst und die zentrale Region (CR) des Gerüsts wenigstens eine nicht spiralförmige Region umfasst.

13. Blutpumpe nach einem der Ansprüche 1 oder 2, ferner umfassend eine Membran, die mit der ausdehnbaren und zusammenklappbaren Ummantelung (290) gekoppelt ist, um einen Fluidkanal zu definieren, der für Blut undurchlässig ist.

14. Blutpumpe nach einem der Ansprüche 1 oder 2, wobei das Gerüst (206) Nitinol umfasst.

15. Blutpumpe nach einem der Ansprüche 1 oder 2, wobei das Gerüst (206) eine polymere Struktur umfasst;
wobei optional ein erstes Durometer der proximalen Laufradregion (IR) des Gerüsts größer ist als ein zweites Durometer der zentralen Region (CR) des Gerüsts.

## Revendications

1. Pompe d'assistance circulatoire de cathéter, comprenant :
une enveloppe dilatable et contractible (290) délimitant une lumière sanguine, l'enveloppe comprenant une région d'hélice (IR) proximale et une région centrale (CR) positionnée distalement par rapport à la région d'hélice proximale ;
un échafaudage (206) s'étendant le long d'au moins une partie de la région d'hélice proximale et de la région centrale de l'enveloppe, l'échafaudage dans la région d'hélice proximale présentant une première rigidité supérieure à une deuxième rigidité de l'échafaudage dans la région centrale, la rigidité servant à assurer un soutien radial à l'enveloppe ;
une hélice proximale (20) disposée au moins partiellement dans la région d'hélice proximale de l'enveloppe.

2. Pompe d'assistance circulatoire selon la revendication 1, dans laquelle l'enveloppe dilatable et contractible (290) comprend une région d'hélice (IR) distale, agencée de façon que la région centrale (CR) de l'enveloppe se trouve entre la région d'hélice distale et la région d'hélice (IR) proximale, et la rigidité est plus grande dans la région d'hélice distale que dans la région centrale.

3. Pompe d'assistance circulatoire selon l'une quelconque des revendications 1 et 2, dans laquelle l'échafaudage (206) comprend une pluralité de bras hélicoïdaux (214) délimitant au moins une région hélicoïdale (213).

4. Pompe d'assistance circulatoire selon la revendication 3, dans laquelle au moins une région hélicoïdale (213) est en chevauchement axial avec l'hélice proximale (202).

5. Pompe d'assistance circulatoire selon la revendication 3, dans laquelle une pluralité de régions hélicoïdales (213) sont en chevauchement axial avec l'hélice proximale (202).

6. Pompe d'assistance circulatoire selon la revendication 3, dans laquelle au moins un bras de la pluralité de bras hélicoïdaux (214) s'étend entre des régions non hélicoïdales adjacentes de l'échafaudage (206).

7. Pompe d'assistance circulatoire selon la revendication 3, dans laquelle au moins une région hélicoïdale (213) s'étend entre des régions non hélicoïdales adjacentes de l'échafaudage (206).

8. Pompe d'assistance circulatoire selon la revendication 1 ou 2, dans laquelle la région d'hélice (IR) proximale de l'échafaudage (206) comprend une pluralité de bras hélicoïdaux (214) délimitant au moins une région hélicoïdale (213).

9. Pompe d'assistance circulatoire selon la revendication 8, dans laquelle au moins une région hélicoïdale (213) est en chevauchement axial avec l'hélice proximale (202).

10. Pompe d'assistance circulatoire selon la revendication 8, dans laquelle une pluralité de régions hélicoïdales (213) sont en chevauchement axial avec l'hélice proximale (202).

11. Pompe d'assistance circulatoire selon la revendication 8, dans laquelle au moins un bras de la pluralité des bras hélicoïdaux (214) s'étend entre des régions non hélicoïdales adjacentes de l'échafaudage (206).

12. Pompe d'assistance circulatoire selon la revendication 3, dans laquelle la région d'hélice (IR) proximale de l'échafaudage (206) comprend au moins une région hélicoïdale (213) et la région centrale (CR) de l'échafaudage comprend au moins une région non hélicoïdale.

13. Pompe d'assistance circulatoire selon quelconque des revendications 1 et 2, comprenant en outre une membrane couplée à l'enveloppe dilatable et contractible (290) pour délimiter un conduit fluidique imperméable au sang.

14. Pompe d'assistance circulatoire selon l'une quelconque des revendications 1 et 2, dans laquelle l'échafaudage (206) comprend du nitinol.

15. Pompe d'assistance circulatoire selon l'une quelconque des revendications 1 et 2, dans laquelle l'échafaudage (206) comprend une structure polymère ;
dans laquelle, éventuellement, une première dureté de la région d'hélice (IR) proximale de l'échafaudage est supérieure à une deuxième dureté de la région centrale (CR) de l'échafaudage.
